# EUROPEAN PATENT APPLICATION

(11) **EP 4 032 904 A1**
(43) Date of publication of application: **27.07.2022**
(21) Application number: 20864350.2
(22) Date of filing: 18.09.2020
(51) Int. Cl.: C07K 16/12, A61K 39/40, A61P 31/04

(54) **ANTI-ALPHA-HEMOLYSIN ANTIBODY AND USE THEREOF**

(30) Priority: 20.09.2019 CN 201910889697
(71) Applicant: Mabwell (Shanghai) Bioscience Co., Ltd., Shanghai 201210 (CN)
(72) Inventor: AN, Maomao, Shanghai 201210 (CN); WANG, Lichun, Shanghai 201210 (CN); GAO, Pan, Shanghai 201210 (CN); LIN, Jian, Shanghai 201210 (CN); JIANG, Yuanying, Shanghai 201210 (CN); SHEN, Hui, Shanghai 201210 (CN); CHEN, Simin, Shanghai 201210 (CN); GUO, Shiyu, Shanghai 201210 (CN); FANG, Wei, Shanghai 201210 (CN)
(74) Representative: Schiweck Weinzierl Koch Patentanwälte Partnerschaft mbB
(86) International application number: PCT/CN2020/116184
(87) International publication number: WO 2021/052461

(57) **Abstract**

Provided are an antibody that binds to Staphylococcus aureus alpha-hemolysin or a fragment thereof, and the use of the antibody or the fragment thereof for preventing or treating Staphylococcus aureus infections. The antibody is obtained through screening by means of the strategies of attenuated immunity and virulent screening of alpha-hemolysin, and the antibody has high affinity to alpha-hemolysin, can effectively block the hemolysis effect of alpha-hemolysin, has proved significant protective or therapeutic effects in the alpha-hemolysin sepsis model, MRSA bacteremia model and MRSA lung infection model, and has a synergistic effect with antibiotics, which is a beneficial supplement to existing antibiotic therapy for Staphylococcus aureus.

Sequence Listing

<110>  MABWELL (SHANGHAI) BIOSCIENCE CO., LTD.

<120>  ANTI-ALPHA-HEMOLYSIN ANTIBODY AND USE THEREOF

<130>  LC20210006P-EP

<150>  CN201910889697.5
<151>  2019-09-20

<160>  87

<170>  PatentIn version 3.3

<210>  1
<211>  7
<212>  PRT
<213>  artificial

<220>
<223>  heavy chain, CDR1

<400>  1

Gly Phe Thr Phe Ser Thr Phe
1               5


<210>  2
<211>  10
<212>  PRT
<213>  artificial

<220>
<223>  heavy chain, CDR1

<400>  2

Gly Phe Thr Phe Ser Thr Phe Gly Met His
1               5                   10


<210>  3
<211>  5
<212>  PRT
<213>  artificial

<220>
<223>  heavy chain, CDR1

<400>  3

Thr Phe Gly Met His
1               5


<210>  4
<211>  6
<212>  PRT
<213>  artificial

<220>

<223>  heavy chain, CDR2

<400>  4

Ser Gly Gly Ser Ser Thr
1               5

<210>  5
<211>  10
<212>  PRT
<213>  artificial

<220>
<223>  heavy chain, CDR2

<400>  5

Tyr Ile Ser Gly Gly Ser Ser Thr Ile Tyr
1               5                   10

<210>  6
<211>  17
<212>  PRT
<213>  artificial

<220>
<223>  heavy chain, CDR2

<400>  6

Tyr Ile Ser Gly Gly Ser Ser Thr Ile Tyr Tyr Ala Asp Thr Val Lys
1               5                   10                  15

Gly

<210>  7
<211>  8
<212>  PRT
<213>  artificial

<220>
<223>  heavy chain, CDR3

<400>  7

Gly Tyr Pro Tyr Gly Leu Asp Tyr
1               5

<210>  8
<211>  11
<212>  PRT
<213>  artificial

<220>
<223>  light chain, CDR1

<400>  8

Lys Ala Ser Gln Asp Ile Asn Trp Tyr Leu Ser
1               5                   10

<210>  9
<211>  7
<212>  PRT
<213>  artificial

<220>
<223>  light chain, CDR2

<400>  9

Arg Gly Asn Arg Leu Val Asp
1               5

<210>  10
<211>  9
<212>  PRT
<213>  artificial

<220>
<223>  light chain, CDR3

<400>  10

Leu Gln Tyr Asp Glu Phe Pro Phe Thr
1               5

<210>  11
<211>  7
<212>  PRT
<213>  artificial

<220>
<223>  light chain, CDR2

<400>  11

Arg Gly Asn Arg Leu Val Glu
1               5

<210>  12
<211>  7
<212>  PRT
<213>  artificial

<220>
<223>  heavy chain, CDR1

<400>  12

Gly Tyr Ser Phe Thr Ser Tyr
1               5

<210>  13
<211>  10

<212>  PRT
<213>  artificial

<220>
<223>  heavy chain, CDR1

<400>  13

Gly Tyr Ser Phe Thr Ser Tyr Trp Met Asn
1               5                   10


<210>  14
<211>  5
<212>  PRT
<213>  artificial

<220>
<223>  heavy chain, CDR1

<400>  14

Ser Tyr Trp Met Asn
1               5


<210>  15
<211>  6
<212>  PRT
<213>  artificial

<220>
<223>  heavy chain, CDR2

<400>  15

His Pro Ser Asp Ser Glu
1               5


<210>  16
<211>  10
<212>  PRT
<213>  artificial

<220>
<223>  heavy chain, CDR2

<400>  16

Met Ile His Pro Ser Asp Ser Glu Thr Arg
1               5                   10


<210>  17
<211>  17
<212>  PRT
<213>  artificial

<220>
<223>  heavy chain, CDR2

<400>  17

Met Ile His Pro Ser Asp Ser Glu Thr Arg Leu Ser Gln Lys Phe Lys
1               5                   10                  15

Asp

<210>  18
<211>  9
<212>  PRT
<213>  artificial

<220>
<223>  heavy chain, CDR3

<400>  18

Phe Asp Trp Asp Arg Ala Met Asp Tyr
1               5

<210>  19
<211>  11
<212>  PRT
<213>  artificial

<220>
<223>  light chain, CDR1

<400>  19

Arg Ala Ser Glu Asn Ile Phe Ser Tyr Leu Ala
1               5                   10

<210>  20
<211>  7
<212>  PRT
<213>  artificial

<220>
<223>  light chain, CDR2

<400>  20

Asn Thr Arg Ser Leu Ala Glu
1               5

<210>  21
<211>  9
<212>  PRT
<213>  artificial

<220>
<223>  light chain, CDR3

<400>  21

Gln His His Tyr Gly Thr Pro Trp Thr
1               5

<210>  22
<211>  7
<212>  PRT
<213>  artificial

<220>
<223>  heavy chain, CDR1

<400>  22

Gly Tyr Thr Phe Ser Glu Tyr
1               5


<210>  23
<211>  10
<212>  PRT
<213>  artificial

<220>
<223>  heavy chain, CDR1

<400>  23

Gly Tyr Thr Phe Ser Glu Tyr Thr Met His
1               5                   10


<210>  24
<211>  5
<212>  PRT
<213>  artificial

<220>
<223>  heavy chain, CDR1

<400>  24

Glu Tyr Thr Met His
1               5


<210>  25
<211>  6
<212>  PRT
<213>  artificial

<220>
<223>  heavy chain, CDR2

<400>  25

Asn Pro Asn Asn Gly Gly
1               5


<210>  26
<211>  10
<212>  PRT
<213>  artificial

<220>
<223>  heavy chain, CDR2

<400>  26

Ser Ile Asn Pro Asn Asn Gly Gly Thr Thr
1               5                   10


<210>  27
<211>  17
<212>  PRT
<213>  artificial

<220>
<223>  heavy chain, CDR2

<400>  27

Ser Ile Asn Pro Asn Asn Gly Gly Thr Thr Tyr Asn Gln Lys Phe Lys
1               5                   10                  15


Gly


<210>  28
<211>  17
<212>  PRT
<213>  artificial

<220>
<223>  heavy chain, CDR2

<400>  28

Ser Ile Asn Pro Asn Gln Gly Gly Thr Thr Tyr Asn Gln Lys Phe Lys
1               5                   10                  15


Gly


<210>  29
<211>  13
<212>  PRT
<213>  artificial

<220>
<223>  heavy chain, CDR3

<400>  29

Thr Arg Asp Tyr Asp Asn Asp Gly Gly Leu Phe Ala Tyr
1               5                   10


<210>  30
<211>  11
<212>  PRT
<213>  artificial

<220>
<223>  light chain, CDR1

<400>  30

Arg Ala Ser Lys Asn Ile Ser Lys Tyr Leu Ala
1               5                   10


<210>  31
<211>  11
<212>  PRT
<213>  artificial

<220>
<223>  light chain, CDR1

<400>  31

Arg Ala Ser Lys Asn Ile Leu Lys Tyr Leu Ala
1               5                   10


<210>  32
<211>  7
<212>  PRT
<213>  artificial

<220>
<223>  light chain, CDR2

<400>  32

Ser Gly Ser Thr Leu Gln Ser
1               5


<210>  33
<211>  9
<212>  PRT
<213>  artificial

<220>
<223>  light chain, CDR3

<400>  33

Gln Gln His Tyr Glu Tyr Pro Phe Thr
1               5


<210>  34
<211>  7
<212>  PRT
<213>  artificial

<220>
<223>  heavy chain, CDR1

<400>  34

Gly Phe Asn Ile Lys Asp Thr

1                   5

<210>  35
<211>  10
<212>  PRT
<213>  artificial

<220>
<223>  heavy chain, CDR1

<400>  35

Gly Phe Asn Ile Lys Asp Thr Tyr Met His
1               5                   10

<210>  36
<211>  5
<212>  PRT
<213>  artificial

<220>
<223>  heavy chain, CDR1

<400>  36

Asp Thr Tyr Met His
1               5

<210>  37
<211>  6
<212>  PRT
<213>  artificial

<220>
<223>  heavy chain, CDR2

<400>  37

Asp Pro Ala Ser Gly Asn
1               5

<210>  38
<211>  10
<212>  PRT
<213>  artificial

<220>
<223>  heavy chain, CDR2

<400>  38

Lys Ile Asp Pro Ala Ser Gly Asn Thr Lys
1               5                   10

<210>  39
<211>  17
<212>  PRT
<213>  artificial

<220>
<223>  heavy chain, CDR2

<400>  39

Lys Ile Asp Pro Ala Ser Gly Asn Thr Lys Tyr Asp Pro Gln Phe Gln
1               5                   10                  15

Gly

<210>  40
<211>  10
<212>  PRT
<213>  artificial

<220>
<223>  heavy chain, CDR3

<400>  40

Pro Tyr Gly Asn Asp Tyr Ala Met Asn Tyr
1               5                   10

<210>  41
<211>  11
<212>  PRT
<213>  artificial

<220>
<223>  light chain, CDR1

<400>  41

Arg Ala Ser Glu Lys Ile Tyr Ser Phe Leu Ala
1               5                   10

<210>  42
<211>  7
<212>  PRT
<213>  artificial

<220>
<223>  light chain, CDR2

<400>  42

Asn Ala Glu Thr Leu Ala Glu
1               5

<210>  43
<211>  9
<212>  PRT
<213>  artificial

<220>
<223>  light chain, CDR3

<400>  43

Gln His His Tyr Gly Thr Pro Tyr Thr
1               5

<210>  44
<211>  117
<212>  PRT
<213>  artificial

<220>
<223>  heavy chain variable region

<400>  44

Asp Val Gln Leu Val Glu Ser Gly Gly Gly Leu Val Gln Pro Gly Gly
1               5                   10                  15

Ser Arg Lys Leu Ser Cys Ala Ala Ser Gly Phe Thr Phe Ser Thr Phe
            20                  25                  30

Gly Met His Trp Val Arg Gln Ala Pro Glu Lys Gly Leu Glu Trp Val
        35                  40                  45

Ala Tyr Ile Ser Gly Gly Ser Ser Thr Ile Tyr Tyr Ala Asp Thr Val
    50                  55                  60

Lys Gly Arg Phe Thr Ile Ser Arg Asp Asn Pro Lys Asn Thr Leu Phe
65                  70                  75                  80

Leu Gln Met Thr Ser Leu Arg Ser Glu Asp Thr Ala Met Tyr Tyr Cys
                85                  90                  95

Ala Ser Gly Tyr Pro Tyr Gly Leu Asp Tyr Trp Gly Gln Gly Thr Ser
            100                 105                 110

Val Thr Val Ser Ser
        115

<210>  45
<211>  351
<212>  DNA
<213>  artificial

<220>
<223>  heavy chain variable region

<400>  45
gatgtgcagc tggtggagtc tgggggaggc ttagtgcagc ctggagggtc ccggaaactc      60

tcctgtgcag cctctggatt cactttcagt acctttggaa tgcactgggt tcgtcaggct     120

ccagagaagg ggctggagtg ggtcgcatac attagtggtg gcagtagtac catctactat     180

gcagacacag tgaagggccg attcaccatc tccagagaca atcccaagaa caccctgttc 240

ctgcaaatga ccagtctaag gtctgaggac acggccatgt attactgtgc aagcggctac 300

ccctatggtt tggactactg gggtcaagga acctcagtca ccgtctcctc a 351

<210> 46
<211> 107
<212> PRT
<213> artificial

<220>
<223> light chain variable region

<400> 46

Asp Ile Asp Met Thr Gln Ser Pro Ser Ser Met Tyr Ala Ser Leu Gly
1               5                   10                  15

Glu Arg Val Thr Ile Thr Cys Lys Ala Ser Gln Asp Ile Asn Trp Tyr
            20                  25                  30

Leu Ser Trp Phe Gln Gln Lys Pro Gly Lys Ser Pro Lys Thr Leu Ile
        35                  40                  45

Tyr Arg Gly Asn Arg Leu Val Asp Gly Val Pro Ser Arg Phe Ser Gly
    50                  55                  60

Ser Gly Ser Gly Gln Asp Tyr Ser Leu Thr Ile Ser Ser Leu Glu Tyr
65                  70                  75                  80

Glu Asp Met Gly Ile Tyr Tyr Cys Leu Gln Tyr Asp Glu Phe Pro Phe
                85                  90                  95

Thr Phe Gly Ser Gly Thr Lys Leu Glu Ile Lys
            100                 105

<210> 47
<211> 321
<212> DNA
<213> artificial

<220>
<223> light chain variable region

<400> 47
gacatcgaca tgacccagtc tccatcttcc atgtatgcat ctctaggaga gagagtcact 60

atcacttgca aggcgagtca ggacattaat tggtatttaa gttggttcca gcagaaacca 120

gggaaatctc ctaagaccct gatctatcgt ggaaacagat tggtagatgg ggtcccatca 180

aggttcagtg gcagtggatc tgggcaagat tattctctca ccatcagcag cctggagtat 240

gaagatatgg gaatttatta ttgtctacag tatgatgagt ttccattcac gttcggctcg 300

gggacaaagt tggaaataaa a 321

<210> 48
<211> 117
<212> PRT
<213> artificial

<220>
<223> heavy chain variable region

<400> 48

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Thr Phe
            20                  25                  30

Gly Met His Trp Val Arg Gln Ala Pro Gly Lys Gly Leu Glu Trp Val
        35                  40                  45

Ser Tyr Ile Ser Gly Gly Ser Ser Thr Ile Tyr Tyr Ala Asp Thr Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Ser Gly Tyr Pro Tyr Gly Leu Asp Tyr Trp Gly Gln Gly Thr Leu
            100                 105                 110

Val Thr Val Ser Ser
        115

<210> 49
<211> 351
<212> DNA
<213> artificial

<220>
<223> heavy chain variable region

<400> 49
gaggtgcagc tggtggagag cgggggggga ctggtgcagc caggaggaag cctgagactg 60

agctgtgccg caagcgggtt cacatttagt acctttggaa tgcactgggt gaggcaggcc 120

cccggcaaag ggctggagtg ggtgtcttat atttccggcg gaagtagcac catatactac 180

gctgatacag tgaagggcag attcaccata agcagggaca acgccaagaa cagcctgtac 240

ctgcagatga acagcctgag agccgaagac accgctgtgt actactgcgc cagcggctac 300

ccctacggcc tggattactg gggacaagga acactggtga ccgtgagcag c 351

<210> 50
<211> 107
<212> PRT
<213> artificial

<220>
<223> light chain variable region

<400> 50

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Ile Asn Trp Tyr
            20                  25                  30

Leu Ser Trp Phe Gln Gln Lys Pro Gly Lys Ala Pro Lys Thr Leu Ile
        35                  40                  45

Tyr Arg Gly Asn Arg Leu Val Glu Gly Val Pro Ser Arg Phe Ser Gly
    50                  55                  60

Ser Gly Ser Gly Gln Asp Tyr Thr Phe Thr Ile Ser Ser Leu Gln Pro
65                  70                  75                  80

Glu Asp Met Ala Thr Tyr Tyr Cys Leu Gln Tyr Asp Glu Phe Pro Phe
                85                  90                  95

Thr Phe Gly Gln Gly Thr Lys Val Glu Ile Lys
            100                 105

<210> 51
<211> 321
<212> DNA
<213> artificial

<220>
<223> light chain variable region

<400> 51
gacattcaga tgacccagag ccccagcagc ctgagcgcca gcgtgggaga cagagtgacc 60

ataacctgca aagccagcca agacatcaac tggtatctgt cctggtttca gcagaagccc 120

ggcaaagccc caaaaaccct catctaccgg ggaaacagac tggtggaagg ggtgcccagc 180

agatttagcg gaagcggcag cggccaggac tacaccttca ccatatcaag cctgcagccc 240

gaagacatgg ccacctacta ctgcctgcag tacgacgaat ttccatttac cttcggacag 300

ggaaccaaag tcgagatcaa a                                              321

<210>  52
<211>  118
<212>  PRT
<213>  artificial

<220>
<223>  heavy chain variable region

<400>  52

Gln Val Gln Leu Gln Gln Pro Gly Ala Glu Leu Val Arg Pro Gly Ala
1               5                   10                  15

Ser Val Lys Leu Ser Cys Lys Ala Ser Gly Tyr Ser Phe Thr Ser Tyr
            20                  25                  30

Trp Met Asn Trp Val Lys Gln Arg Pro Gly Gln Gly Leu Glu Trp Ile
        35                  40                  45

Gly Met Ile His Pro Ser Asp Ser Glu Thr Arg Leu Ser Gln Lys Phe
    50                  55                  60

Lys Asp Lys Ala Thr Leu Thr Val Asp Lys Ser Ser Ser Thr Ala Tyr
65                  70                  75                  80

Met Gln Leu Ser Ser Pro Thr Ser Glu Asp Ser Ala Val Tyr Tyr Cys
                85                  90                  95

Thr Arg Phe Asp Trp Asp Arg Ala Met Asp Tyr Trp Gly Gln Gly Thr
            100                 105                 110

Ser Val Thr Val Ser Ser
        115

<210>  53
<211>  354
<212>  DNA
<213>  artificial

<220>
<223>  heavy chain variable region

<400>  53
caggtccaac tgcagcagcc tggggctgag ctggtgaggc ctggagcttc agtgaagctg      60

tcctgcaagg cttctggcta ctccttcacc agctactgga tgaactgggt gaagcagagg     120

cctggacaag gccttgagtg gattggcatg attcatcctt ccgatagtga aactaggtta     180

agtcagaagt tcaaggacaa ggccacattg actgtagaca aatcctccag cacagcctac     240

atgcaactca gcagcccgac atctgaggac tctgcggtct attactgtac aagattcgac     300

tgggaccggg ctatggacta ctggggtcaa ggaacctcag tcaccgtctc ctca 354

<210> 54
<211> 107
<212> PRT
<213> artificial

<220>
<223> light chain variable region

<400> 54

Asp Ile Gln Met Thr Gln Ser Pro Ala Ser Leu Ser Ala Ser Val Gly
1               5                   10                  15

Glu Thr Val Thr Ile Thr Cys Arg Ala Ser Glu Asn Ile Phe Ser Tyr
            20                  25                  30

Leu Ala Trp Tyr Gln Gln Lys Gln Gly Lys Ser Pro Gln Leu Leu Val
        35                  40                  45

Tyr Asn Thr Arg Ser Leu Ala Glu Gly Val Pro Ser Arg Phe Ser Gly
    50                  55                  60

Ser Gly Ser Gly Thr Gln Phe Ser Leu Lys Ile Asn Ser Leu Gln Pro
65                  70                  75                  80

Glu Asp Phe Gly Thr Tyr Tyr Cys Gln His His Tyr Gly Thr Pro Trp
                85                  90                  95

Thr Phe Gly Gly Gly Thr Lys Leu Glu Ile Lys
            100                 105

<210> 55
<211> 321
<212> DNA
<213> artificial

<220>
<223> light chain variable region

<400> 55
gacatccaga tgactcagtc tccagcctcc ctatctgcat ctgtgggaga aactgtcacc 60

atcacatgtc gagcaagtga gaatattttc agttatttag catggtatca acagaaacag 120

ggaaaatctc ctcagctcct ggtctataat acaagatcct tagcagaagg tgtgccatca 180

aggttcagtg gcagtggatc aggcacacag ttttctctga agatcaacag cctgcagcct 240

gaagattttg ggacttatta ctgtcaacat cattatggta ctccgtggac gttcggtgga 300

ggcaccaagc tggaaatcaa a 321

<210>  56
<211>  118
<212>  PRT
<213>  artificial

<220>
<223>  heavy chain variable region

<400>  56

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ser Phe Thr Ser Tyr
            20                  25                  30

Trp Met Asn Trp Val Arg Gln Ala Pro Gly Gln Gly Leu Glu Trp Met
        35                  40                  45

Gly Met Ile His Pro Ser Asp Ser Glu Thr Arg Leu Ser Gln Lys Phe
    50                  55                  60

Lys Asp Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Phe Asp Trp Asp Arg Ala Met Asp Tyr Trp Gly Gln Gly Thr
            100                 105                 110

Leu Val Thr Val Ser Ser
        115


<210>  57
<211>  354
<212>  DNA
<213>  artificial

<220>
<223>  heavy chain variable region

<400>  57
caggtccaac tcgtccaaag cggcgcagaa gtcaaaaagc ccggcgcatc agtcaaagtt      60

agctgcaagg ccagcggcta cagcttcaca tcatactgga tgaactgggt gcggcaagcc     120

cccggccaag gtctcgaatg gatgggaatg atccacccca gcgacagcga gaccaggctg     180

agccagaaat ttaaagacag agtcaccatg accagagaca cctccacctc aaccgtctat     240

atggaactga gcagcctcag aagcgaggac accgccgtgt attactgcgc ccggttcgac     300

tgggacagag ccatggacta ctggggccaa ggcaccctcg ttaccgtgag cagc           354

<210>  58
<211>  107
<212>  PRT
<213>  artificial

<220>
<223>  light chain variable region

<400>  58

Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Asn Ile Phe Ser Tyr
            20                  25                  30

Leu Ala Trp Tyr Gln Gln Lys Pro Gly Lys Ala Pro Lys Leu Leu Ile
        35                  40                  45

Tyr Asn Thr Arg Ser Leu Ala Glu Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Gly Thr Pro Trp
                85                  90                  95

Thr Phe Gly Gln Gly Thr Lys Leu Glu Ile Lys
            100                 105


<210>  59
<211>  321
<212>  DNA
<213>  artificial

<220>
<223>  light chain variable region

<400>  59
gacattcaga tgacccagag ccccagcagc ctgagcgcca gcgtgggaga cagagtgacc      60

ataacctgca gagccagcga gaacatattc tcatacctcg cctggtacca gcagaaaccc     120

ggcaaagccc caaaactgct catctacaac acaagaagcc tggctgaagg agtgcccagc     180

agattcagcg ggtcaggcag cggcaccgac ttcaccctga ccatcagcag cctgcaacca     240

gaagacttcg ccacctacta ctgccaacac cactacggca ccccctggac cttcggacaa     300

ggcaccaaac tcgagatcaa a                                               321


<210>  60

<211>  122
<212>  PRT
<213>  artificial

<220>
<223>  heavy chain variable region

<400>  60

Glu Val His Leu Gln Gln Ser Gly Pro Glu Leu Met Lys Pro Gly Ala
1               5                   10                  15

Ser Val Lys Ile Ser Cys Lys Thr Ser Gly Tyr Thr Phe Ser Glu Tyr
            20                  25                  30

Thr Met His Trp Val Lys Gln Ser His Gly Lys Ser Leu Glu Trp Ile
        35                  40                  45

Gly Ser Ile Asn Pro Asn Asn Gly Gly Thr Thr Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Arg Ser Leu Thr Ser Glu Asp Ser Ala Val Tyr Asn Cys
                85                  90                  95

Ala Arg Thr Arg Asp Tyr Asp Asn Asp Gly Gly Leu Phe Ala Tyr Trp
            100                 105                 110

Gly Gln Gly Thr Leu Val Thr Val Ser Ala
        115                 120


<210>  61
<211>  366
<212>  DNA
<213>  artificial

<220>
<223>  heavy chain variable region

<400>  61
gaggtccacc tgcaacagtc tggacctgag ctgatgaagc ctggggcttc agtgaagata      60

tcctgcaaga cttctggata cacattcagt gaatacacca tgcactgggt gaagcagagc     120

catggaaaga gccttgagtg gattggaagt attaatccta acaatggtgg tactacctac     180

aaccagaagt tcaagggcaa ggccacattg actgtagaca agtcctccag cacagcctac     240

atggagctcc gcagcctgac atctgaggat tctgcagtct ataactgtgc aagaactagg     300

gactatgata acgacggggg tctttttgct tactggggcc aagggactct ggtcactgtc     360

tctgca                                                                366

<210>  62
<211>  107
<212>  PRT
<213>  artificial

<220>
<223>  light chain variable region

<400>  62

Asp Val Gln Ile Thr Gln Ser Pro Ser Tyr Leu Ala Ala Ser Pro Gly
1               5                   10                  15

Glu Thr Ile Thr Ile Asn Cys Arg Ala Ser Lys Asn Ile Ser Lys Tyr
            20                  25                  30

Leu Ala Trp Tyr Gln Glu Lys Pro Gly Lys Thr Asn Lys Leu Leu Ile
        35                  40                  45

Tyr Ser Gly Ser Thr Leu Gln Ser Gly Ile Pro Ser Arg Phe Ser Gly
    50                  55                  60

Asn Arg Ser Gly Thr Asp Phe Thr Leu Thr Ile Ser Ser Leu Glu Pro
65                  70                  75                  80

Glu Asp Phe Ala Met Tyr Tyr Cys Gln Gln His Tyr Glu Tyr Pro Phe
                85                  90                  95

Thr Phe Gly Gly Gly Thr Lys Leu Glu Ile Lys
            100                 105


<210>  63
<211>  321
<212>  DNA
<213>  artificial

<220>
<223>  light chain variable region

<400>  63
gatgtccaga taacccagtc tccatcttat cttgctgcat ctcctggaga aaccattact      60

attaattgca gggcaagtaa gaacattagc aaatatttag cctggtatca agagaaacct     120

gggaaaacta ataagcttct tatctactct ggatccactt tgcaatctgg aattccatca     180

aggttcagtg gcaatagatc tggtacagat ttcactctca ccatcagtag cctggagcct     240

gaagattttg caatgtatta ctgtcaacaa cattatgaat acccgttcac gttcggaggg     300

gggaccaagc tggaaataaa a                                               321


<210>  64

<211>  122
<212>  PRT
<213>  artificial

<220>
<223>  heavy chain variable region

<400>  64

Gln Val Gln Leu Val Gln Ser Gly Ala Glu Val Lys Lys Pro Gly Ala
1               5                   10                  15

Ser Val Lys Val Ser Cys Lys Thr Ser Gly Tyr Thr Phe Ser Glu Tyr
            20                  25                  30

Thr Met His Trp Val Arg Gln Ala Pro Gly Gln Arg Leu Glu Trp Met
        35                  40                  45

Gly Ser Ile Asn Pro Asn Gln Gly Gly Thr Thr Tyr Asn Gln Lys Phe
    50                  55                  60

Lys Gly Arg Val Thr Ile Thr Val Asp Lys Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Thr Arg Asp Tyr Asp Asn Asp Gly Gly Leu Phe Ala Tyr Trp
            100                 105                 110

Gly Gln Gly Thr Leu Val Thr Val Ser Ser
        115                 120


<210>  65
<211>  366
<212>  DNA
<213>  artificial

<220>
<223>  heavy chain variable region

<400>  65
caggtccaac tcgtccaaag cggcgcagaa gtcaaaaagc ccggcgcatc agtcaaagtt      60

agctgcaaga catccggcta caccttcagc gagtacacca tgcactgggt gagacaagcc     120

ccaggccaaa gactggagtg gatgggaagc atcaacccca accaaggcgg caccacctac     180

aaccaaaaat tcaagggcag agtgacaatt accgtggaca agagcgccag caccgcctac     240

atggagctgt ctagcctgag aagcgaggac accgccgtgt actattgcgc cagaaccaga     300

gactacgaca acgatggagg actgttcgcc tattggggcc agggaaccct cgtgaccgtg     360

agcagc                                                                366

<210>  66
<211>  107
<212>  PRT
<213>  artificial

<220>
<223>  light chain variable region

<400>  66

Asp Val Gln Ile Thr Gln Ser Pro Ser Phe Leu Ser Ala Ser Val Gly
1               5                   10                  15

Asp Arg Val Thr Ile Thr Cys Arg Ala Ser Lys Asn Ile Leu Lys Tyr
            20                  25                  30

Leu Ala Trp Tyr Gln Gln Lys Pro Gly Lys Ala Pro Lys Leu Leu Ile
        35                  40                  45

Tyr Ser Gly Ser Thr Leu Gln Ser Gly Val Pro Ser Arg Phe Ser Gly
    50                  55                  60

Ser Arg Ser Gly Thr Glu Phe Thr Leu Thr Ile Ser Ser Leu Gln Pro
65                  70                  75                  80

Glu Asp Phe Ala Thr Tyr Tyr Cys Gln Gln His Tyr Glu Tyr Pro Phe
                85                  90                  95

Thr Phe Gly Gln Gly Thr Lys Leu Glu Ile Lys
            100                 105

<210>  67
<211>  321
<212>  DNA
<213>  artificial

<220>
<223>  light chain variable region

<400>  67
gacgtgcaga ttacccaaag ccccagcttc ctgtccgcca gcgtgggcga cagagtgaca      60

attacatgca gagccagcaa gaacatactg aagtacctgg catggtacca acaaaaaccc     120

ggcaaggccc ccaaactgct catctactcc ggcagtaccc tgcagagcgg cgtgcccagc     180

agattcagcg gaagcagaag cggcaccgag ttcactctga ccatcagcag cctccaacca     240

gaggacttcg ccacctacta ctgccagcag cactacgaat accccttcac cttcggccag     300

ggcaccaagc tggagatcaa a                                               321

<210>  68

<211>  119
<212>  PRT
<213>  artificial

<220>
<223>  heavy chain variable region

<400>  68

Glu Val Gln Leu Gln Gln Ser Gly Ala Glu Leu Val Lys Pro Gly Ala
1               5                   10                  15

Ser Val Thr Leu Ser Cys Thr Val Ser Gly Phe Asn Ile Lys Asp Thr
            20                  25                  30

Tyr Met His Trp Val Lys Gln Arg Pro Glu Gln Gly Leu Glu Trp Ile
        35                  40                  45

Gly Lys Ile Asp Pro Ala Ser Gly Asn Thr Lys Tyr Asp Pro Gln Phe
    50                  55                  60

Gln Gly Lys Ala Thr Ile Thr Ala Asp Thr Ser Ser Asn Thr Ala Tyr
65                  70                  75                  80

Leu His Leu Ser Ser Leu Thr Ser Glu Asp Ser Ala Val Tyr Phe Cys
                85                  90                  95

Ala Ser Pro Tyr Gly Asn Asp Tyr Ala Met Asn Tyr Trp Gly Gln Gly
            100                 105                 110

Thr Ser Val Thr Val Ser Ser
        115

<210>  69
<211>  357
<212>  DNA
<213>  artificial

<220>
<223>  heavy chain variable region

<400>  69
gaggttcagc tgcagcagtc tggggcagaa cttgtgaagc caggggcctc agtcacgttg      60

tcctgcacag tttctggctt caacattaaa gacacctata tgcactgggt gaaacagagg     120

cctgaacagg gcctggagtg gattggaaag attgatcctg cgagtggtaa tactaaatat     180

gacccgcagt tccagggcaa ggccactata acagcagaca catcctccaa cacagcctac     240

ctgcacctca gcagcctgac atctgaggac agtgccgtct atttctgtgc tagcccctat     300

ggtaacgact atgctatgaa ctactgggga caaggaacct cagtcaccgt ctcctca        357

<210>  70
<211>  107
<212>  PRT
<213>  artificial

<220>
<223>  light chain variable region

<400>  70

Asp Ile Gln Met Thr Gln Ser Pro Ala Ser Leu Ser Ala Ser Val Gly
1               5                   10                  15

Glu Thr Val Thr Ile Thr Cys Arg Ala Ser Glu Lys Ile Tyr Ser Phe
            20                  25                  30

Leu Ala Trp Tyr Gln Gln Lys Gln Glu Lys Ser Pro Gln Leu Leu Val
        35                  40                  45

Tyr Asn Ala Glu Thr Leu Ala Glu Gly Val Pro Ser Arg Phe Ser Gly
    50                  55                  60

Thr Gly Ser Gly Ile Gln Phe Ser Leu Lys Ile Ile Ser Leu Gln Pro
65                  70                  75                  80

Glu Asp Phe Gly Ile Tyr Tyr Cys Gln His His Tyr Gly Thr Pro Tyr
                85                  90                  95

Thr Phe Gly Gly Gly Thr Lys Leu Glu Ile Lys
            100                 105

<210>  71
<211>  321
<212>  DNA
<213>  artificial

<220>
<223>  light chain variable region

<400>  71
gacatccaga tgactcagtc tccagcctcc ctatctgcat ctgtgggaga aactgtcacc      60

atcacatgtc gagcaagtga gaaaatttac agttttttag catggtatca gcagaaacag      120

gaaaaatctc ctcaactcct ggtctataat gcagaaacct tagcagaagg tgtgccatca      180

aggttcagtg gcactggatc gggcatccag ttttctctga agattatcag cctgcagcct      240

gaagattttg ggatttatta ctgtcaacat cattatggta ctccgtacac gttcggaggg      300

gggaccaagt tggaaataaa a                                                321

<210>  72
<211>  119
<212>  PRT

<213>  artificial

<220>
<223>  heavy chain variable region

<400>  72

Glu Val Gln Leu Val Gln Ser Gly Ala Glu Val Lys Lys Pro Gly Ala
1               5                   10                  15

Thr Val Lys Ile Ser Cys Lys Val Ser Gly Phe Asn Ile Lys Asp Thr
            20                  25                  30

Tyr Met His Trp Val Gln Gln Ala Pro Gly Lys Gly Leu Glu Trp Met
        35                  40                  45

Gly Lys Ile Asp Pro Ala Ser Gly Asn Thr Lys Tyr Asp Pro Gln Phe
    50                  55                  60

Gln Gly Arg Val Thr Ile Thr Ala Asp Thr Ser Thr Asn Thr Ala Tyr
65                  70                  75                  80

Met Glu Leu Ser Ser Leu Arg Ser Glu Asp Thr Ala Val Tyr Tyr Cys
                85                  90                  95

Ala Thr Pro Tyr Gly Asn Asp Tyr Ala Met Asn Tyr Trp Gly Gln Gly
            100                 105                 110

Thr Leu Val Thr Val Ser Ser
        115

<210>  73
<211>  357
<212>  DNA
<213>  artificial

<220>
<223>  heavy chain variable region

<400>  73
gaggtgcagc tggtgcagag cggagcagag gtgaagaagc caggggccac agtgaagata      60

agctgtaagg tgagcggatt caacattaag gacacatata tgcactgggt gcagcaggca     120

cccggcaaag gactggagtg gatgggaaag atcgaccccg ccagtggcaa taccaagtac     180

gacccccagt tccagggccg agtgaccatc accgcagaca ccagcaccaa cacagcctac     240

atggagctga gcagcctccg cagcgaagac acagccgtgt actactgcgc caccccctat      300

ggcaacgact acgctatgaa ttactggggc cagggaacac tggtcaccgt gtccagc        357

<210>  74
<211>  107

<212>  PRT
<213>  artificial

<220>
<223>  light chain variable region

<400>  74

Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Lys Ile Tyr Ser Phe
            20                  25                  30

Leu Ala Trp Tyr Gln Gln Lys Pro Gly Lys Ala Pro Lys Leu Leu Leu
        35                  40                  45

Tyr Asn Ala Glu Thr Leu Ala Glu Gly Val Pro Ser Arg Phe Ser Gly
    50                  55                  60

Ser Gly Ser Gly Ile Asp Phe Thr Leu Thr Ile Ser Ser Leu Gln Pro
65                  70                  75                  80

Glu Asp Phe Ala Thr Tyr Tyr Cys Gln His His Tyr Gly Thr Pro Tyr
                85                  90                  95

Thr Phe Gly Gly Gly Thr Lys Val Glu Ile Lys
            100                 105

<210>  75
<211>  321
<212>  DNA
<213>  artificial

<220>
<223>  light chain variable region

<400>  75
gacattcaga tgacccagag ccccagcagc ctgagcgcca gcgtgggaga cagagtgacc      60

ataacctgca gagccagcga gaaaatatat agctttctgg cctggtatca gcagaagccc     120

ggaaaagccc caaaactgct gctgtacaac gcagaaaccc tggcagaggg agtgcccagc     180

agattcagcg gatcaggaag cggcatcgac ttcaccctga ccatcagcag cctgcaaccc     240

gaagacttcg ccacctacta ctgccaacac cactacggca ccccatacac cttcggagga     300

ggcacaaagg tcgaaatcaa a                                               321

<210>  76
<211>  107
<212>  PRT
<213>  artificial

<220>
<223>  light chain variable region

<400>  76

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Ile Asn Trp Tyr
            20                  25                  30

Leu Ser Trp Phe Gln Gln Lys Pro Gly Lys Ala Pro Lys Thr Leu Ile
        35                  40                  45

Tyr Arg Gly Asn Arg Leu Val Asp Gly Val Pro Ser Arg Phe Ser Gly
    50                  55                  60

Ser Gly Ser Gly Gln Asp Tyr Thr Phe Thr Ile Ser Ser Leu Gln Pro
65                  70                  75                  80

Glu Asp Met Ala Thr Tyr Tyr Cys Leu Gln Tyr Asp Glu Phe Pro Phe
                85                  90                  95

Thr Phe Gly Gln Gly Thr Lys Val Glu Ile Lys
            100                 105

<210>  77
<211>  321
<212>  DNA
<213>  artificial

<220>
<223>  light chain variable region

<400>  77
gacattcaga tgacccagag ccccagcagc ctgagcgcca gcgtgggaga cagagtgacc      60

ataacctgca aagccagcca agacatcaac tggtatctgt cctggtttca gcagaagccc     120

ggcaaagccc caaaaaccct catctaccgg ggaaacagac tggtggacgg agtgcccagc     180

agattcagcg gaagcggcag cgggcaagac tacaccttca ccatatcaag cctgcagccc     240

gaagacatgg ccacctacta ctgcctgcag tacgacgaat tcccctttac cttcggccaa     300

gggaccaagg tggagatcaa g                                               321

<210>  78
<211>  294
<212>  PRT
<213>  Staphylococcus aureus

<400>  78

Met Ala Asp Ser Asp Ile Asn Ile Lys Thr Gly Thr Thr Asp Ile Gly

1               5                   10                  15

Ser Asn Thr Thr Val Lys Thr Gly Asp Leu Val Thr Tyr Asp Lys Glu
            20                  25                  30

Asn Gly Met His Lys Lys Val Phe Tyr Ser Phe Ile Asp Asp Lys Asn
        35                  40                  45

His Asn Lys Lys Leu Leu Val Ile Arg Thr Lys Gly Thr Ile Ala Gly
    50                  55                  60

Gln Tyr Arg Val Tyr Ser Glu Glu Gly Ala Asn Lys Ser Gly Leu Ala
65                  70                  75                  80

Trp Pro Ser Ala Phe Lys Val Gln Leu Gln Leu Pro Asp Asn Glu Val
                85                  90                  95

Ala Gln Ile Ser Asp Tyr Tyr Pro Arg Asn Ser Ile Asp Thr Lys Glu
            100                 105                 110

Tyr Met Ser Thr Leu Thr Tyr Gly Phe Asn Gly Asn Val Thr Gly Asp
        115                 120                 125

Asp Thr Gly Lys Ile Gly Gly Leu Ile Gly Ala Asn Val Ser Ile Gly
    130                 135                 140

His Thr Leu Lys Tyr Val Gln Pro Asp Phe Lys Thr Ile Leu Glu Ser
145                 150                 155                 160

Pro Thr Asp Lys Lys Val Gly Trp Lys Val Ile Phe Asn Asn Met Val
                165                 170                 175

Asn Gln Asn Trp Gly Pro Tyr Asp Arg Asp Ser Trp Asn Pro Val Tyr
            180                 185                 190

Gly Asn Gln Leu Phe Met Lys Thr Arg Asn Gly Ser Met Lys Ala Ala
        195                 200                 205

Asp Asn Phe Leu Asp Pro Asn Lys Ala Ser Ser Leu Leu Ser Ser Gly
    210                 215                 220

Phe Ser Pro Asp Phe Ala Thr Val Ile Thr Met Asp Arg Lys Ala Ser
225                 230                 235                 240

Lys Gln Gln Thr Asn Ile Asp Val Ile Tyr Glu Arg Val Arg Asp Asp
                245                 250                 255

Tyr Gln Leu His Trp Thr Ser Thr Asn Trp Lys Gly Thr Asn Thr Lys
            260                 265                 270

Asp Lys Trp Thr Asp Arg Ser Ser Glu Arg Tyr Lys Ile Asp Trp Glu
        275                 280                 285

Lys Glu Glu Met Thr Asn
    290

<210>  79
<211>  891
<212>  DNA
<213>  Staphylococcus aureus

<400>  79
catatggcag acagcgacat taacattaaa accggcacca ccgatatcgg aagcaatacc      60

accgtaaaaa ccggagatct ggtcacctat gataaagaaa acggcatgct gaaaaaggtg     120

ttttatagct ttatcgacga taaaaaccac aataaaaagc tgttagtgat ccgcaccaaa     180

ggtacaatcg caggtcaata cagagtttat agcgaagaag gtgccaataa aagcggtctg     240

gcatggccga gcgcatttaa agtgcagctg cagctgccgg acaatgaagt tgcacagata     300

agcgactatt atccacgtaa tagtattgac acaaaggaat atatgagcac cctgacctat     360

ggttttaacg gtaatgttac cggtgatgac accggtaaga ttgggggatt aattggtgca     420

aatgtttcta ttggtcacac cctgaaatat gttcaaccgg attttaagac catcctggaa     480

tctccgacag ataaaaaggt ggggtggaaa gttatcttta ataatatggt taaccagaac     540

tggggcccgt atgatcgcga tagctggaat ccagtttatg gtaatcagct gttcatgaaa     600

acacgcaatg gcagcatgaa agcagcagat aactttctgg acccgaataa agcaagcagc     660

ctgctgagca gcggtttttc accggacttt gcaaccgtta tcaccatgga tcgcaaagcc     720

tcaaaacagc agaccaacat tgatgttatc tacgaaagag tacgcgatga ttaccagtta     780

cattggacaa gcaccaattg gaaaggaacc aataccaaag acaaatggac cgatagaagc     840

agcgaacggt ataaaattga ttgggagaaa gaagaaatga ccaatctcga g              891

<210>  80
<211>  294
<212>  PRT
<213>  artificial

<220>
<223>  alpha-hemolysin mutant

<400>  80

Met Ala Asp Ser Asp Ile Asn Ile Lys Thr Gly Thr Thr Asp Ile Gly
1               5                   10                  15

Ser Asn Thr Thr Val Lys Thr Gly Asp Leu Val Thr Tyr Asp Lys Glu
                20                  25                  30

Asn Gly Met Leu Lys Lys Val Phe Tyr Ser Phe Ile Asp Asp Lys Asn
            35                  40                  45

His Asn Lys Lys Leu Leu Val Ile Arg Thr Lys Gly Thr Ile Ala Gly
    50                  55                  60

Gln Tyr Arg Val Tyr Ser Glu Glu Gly Ala Asn Lys Ser Gly Leu Ala
65                  70                  75                  80

Trp Pro Ser Ala Phe Lys Val Gln Leu Gln Leu Pro Asp Asn Glu Val
                85                  90                  95

Ala Gln Ile Ser Asp Tyr Tyr Pro Arg Asn Ser Ile Asp Thr Lys Glu
            100                 105                 110

Tyr Met Ser Thr Leu Thr Tyr Gly Phe Asn Gly Asn Val Thr Gly Asp
        115                 120                 125

Asp Thr Gly Lys Ile Gly Gly Leu Ile Gly Ala Asn Val Ser Ile Gly
    130                 135                 140

His Thr Leu Lys Tyr Val Gln Pro Asp Phe Lys Thr Ile Leu Glu Ser
145                 150                 155                 160

Pro Thr Asp Lys Lys Val Gly Trp Lys Val Ile Phe Asn Asn Met Val
                165                 170                 175

Asn Gln Asn Trp Gly Pro Tyr Asp Arg Asp Ser Trp Asn Pro Val Tyr
            180                 185                 190

Gly Asn Gln Leu Phe Met Lys Thr Arg Asn Gly Ser Met Lys Ala Ala
        195                 200                 205

Asp Asn Phe Leu Asp Pro Asn Lys Ala Ser Ser Leu Leu Ser Ser Gly
    210                 215                 220

Phe Ser Pro Asp Phe Ala Thr Val Ile Thr Met Asp Arg Lys Ala Ser
225                 230                 235                 240

Lys Gln Gln Thr Asn Ile Asp Val Ile Tyr Glu Arg Val Arg Asp Asp
                245                 250                 255

Tyr Gln Leu His Trp Thr Ser Thr Asn Trp Lys Gly Thr Asn Thr Lys
            260                 265                 270

Asp Lys Trp Thr Asp Arg Ser Ser Glu Arg Tyr Lys Ile Asp Trp Glu
        275                 280                 285

Lys Glu Glu Met Thr Asn
    290

<210>  81
<211>  891
<212>  DNA
<213>  artificial

<220>
<223>  alpha-hemolysin mutant

<400>  81
catatggcag acagcgacat taacattaaa accggcacca ccgatatcgg aagcaatacc      60

accgtaaaaa ccggagatct ggtcacctat gataaagaaa acggcatgct gaaaaaggtg     120

ttttatagct ttatcgacga taaaaaccac aataaaaagc tgttagtgat ccgcaccaaa     180

ggtacaatcg caggtcaata cagagtttat agcgaagaag gtgccaataa aagcggtctg     240

gcatggccga gcgcatttaa agtgcagctg cagctgccgg acaatgaagt tgcacagata     300

agcgactatt atccacgtaa tagtattgac acaaaggaat atatgagcac cctgacctat     360

ggttttaacg gtaatgttac cggtgatgac accggtaaga ttgggggatt aattggtgca     420

aatgtttcta ttggtcacac cctgaaatat gttcaaccgg attttaagac catcctggaa     480

tctccgacag ataaaaaggt ggggtggaaa gttatcttta ataatatggt taaccagaac     540

tggggcccgt atgatcgcga tagctggaat ccagtttatg gtaatcagct gttcatgaaa     600

acacgcaatg gcagcatgaa agcagcagat aactttctgg acccgaataa agcaagcagc     660

ctgctgagca gcggtttttc accggacttt gcaaccgtta tcaccatgga tcgcaaagcc     720

tcaaaacagc agaccaacat tgatgttatc tacgaaagag tacgcgatga ttaccagtta     780

cattggacaa gcaccaattg gaaagggacc aataccaaag acaaatggac cgatagaagc     840

agcgaacggt ataaaattga ttgggagaaa gaagaaatga ccaatctcga g              891

<210>  82
<211>  129
<212>  PRT
<213>  artificial

<220>
<223>  heavy chain variable region

<400>  82

Glu Val Gln Met Val Gln Ser Gly Ala Glu Val Lys Lys Pro Gly Glu
1               5                   10                  15

Pro Leu Lys Ile Ser Cys Lys Gly Ser Gly Tyr Lys Phe Gly Thr His
            20                  25                  30

Trp Ile Gly Trp Val Arg Gln Arg Pro Gly Lys Gly Leu Glu Trp Met
        35                  40                  45

Gly Ile Ile His Pro Ala Asp Ser Glu Thr Lys Tyr Ser Pro Ser Phe
    50                  55                  60

Gln Gly Gln Val Ser Phe Ser Ala Asp Lys Ser Ser Asn Thr Ala Tyr
65                  70                  75                  80

Leu His Trp Ser Thr Leu Arg Ala Ser Asp Thr Ala Met Tyr Tyr Cys
                85                  90                  95

Ala Arg Arg Ser Gly Ser Ser Ser Trp Tyr Ala Leu Asp Phe Trp Gly
            100                 105                 110

Gln Gly Thr Met Val Thr Val Ser Ser Ala Ser Thr Lys Gly Pro Ser
        115                 120                 125

Val

<210> 83
<211> 123
<212> PRT
<213> artificial

<220>
<223> light chain variable region

<400> 83

Gln Ser Val Leu Thr Gln Ser Pro Ser Ala Ser Gly Thr Pro Gly Gln
1               5                   10                  15

Arg Val Thr Ile Ser Cys Ser Gly Gly Ser Ser Asn Ile Gly Ser Asn
            20                  25                  30

Thr Val Asn Trp Tyr Gln Gln Phe Pro Gly Ala Ala Pro Lys Leu Leu
        35                  40                  45

Ile Tyr Thr Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Thr Trp Asp Asp Ser Leu

85                  90                  95

Asn Gly Leu Tyr Val Phe Gly Thr Gly Thr Lys Val Thr Val Leu Gly
            100                 105                 110

Gln Pro Lys Ala Asn Pro Thr Val Thr Leu Phe
        115                 120

<210>  84
<211>  122
<212>  PRT
<213>  artificial

<220>
<223>  heavy chain variable region

<400>  84

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser His
            20                  25                  30

Asp Met His Trp Val Arg Gln Ala Thr Gly Lys Gly Leu Glu Trp Val
        35                  40                  45

Ser Gly Ile Gly Thr Ala Gly Asp Thr Tyr Tyr Pro Asp Ser Val Lys
    50                  55                  60

Gly Arg Phe Thr Ile Ser Arg Glu Asn Ala Lys Asn Ser Leu Tyr Leu
65                  70                  75                  80

Gln Met Asn Ser Leu Arg Ala Gly Asp Thr Ala Val Tyr Tyr Cys Ala
                85                  90                  95

Arg Asp Arg Tyr Ser Pro Thr Gly His Tyr Tyr Gly Met Asp Val Trp
            100                 105                 110

Gly Gln Gly Thr Thr Val Thr Val Ser Ser
        115                 120

<210>  85
<211>  106
<212>  PRT
<213>  artificial

<220>
<223>  light chain variable region

<400>  85

Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Ser Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Lys Gln Tyr Ala Asp Tyr Trp Thr
                85                  90                  95

Phe Gly Gln Gly Thr Lys Val Glu Ile Lys
            100                 105

<210>  86
<211>  330
<212>  PRT
<213>  artificial

<220>
<223>  heavy chain constant region

<400>  86

<210> 87
<211> 107
<212> PRT
<213> artificial

<220>
<223>  light chain constant region

<400>  87

## Description

The present application claims the priority benefit of Chinese Patent Application No. CN201910889697.5 filed on September 20, 2019, which is hereby incorporated by reference in its entirety.

### TECHNICAL FIELD

The present invention relates to the field of antibody drugs, in particular to an anti-alpha-hemolysin antibody and pharmaceutical application thereof.

### BACKGROUND OF THE PRESENT INVENTION

Staphylococcus aureus, belonging to Staphylococcus, is an important gram-positive pathogenic bacterium, and is the most important G+ pathogenic bacterium in human. Staphylococcus aureus can cause local infections, such as pyogenic infection, pneumonia, pseudomembranous enteritis, pericarditis, etc., as well as systemic infections, such as septicemia, sepsis, etc. Data available on the China Antimicrobial Surveillance Network showed Staphylococcus aureus ranked 4th among pathogenic bacteria detected in hospitals and 1st among G+ bacteria detected in hospitals.

Staphylococcus aureus releases a large amount of toxins to destroy tissue cells during a process of infection in human body, and inhibits immune cells in the body from eliminating pathogens. At present, beta-lactam antibiotics are mainly adopted clinically to treat Staphylococcus aureus infection. However, antibiotics only inhibit or kill bacteria, but do nothing to the toxins released by bacteria. On the contrary, bacteria release more toxins under the pressure of antibiotics, and bacteria killed by antibiotics release toxins upon lysis also. When a large amount of toxins enter blood, the host immune system will be excessively activated to release excessive inflammatory factors, thereby forming sepsis.

Moreover, in the process of fighting with human beings, Staphylococcus aureus has become less and less sensitive to beta-lactam antibiotics, for example, methicillin-resistant Staphylococcus aureus (MRSA) is separated more and more at present. Data available on the China Antimicrobial Surveillance Network showed in 2016 the isolating rate of MRSA was even as high as 38.9%. For MRSA, therapeutic drugs clinically mainly include a limited number of ones, for example, vancomycin and linezolid, which however are typical representatives of drugs that can result in "super-resistant" bacteria infecting humans. Therefore, clinical MRSA infection becomes increasing serious, and the selection of antibacterial agents is very limited, both of which lead to clinical treatment failure in patients and a high mortality rate of patients. Even infection caused by VRSA (vancomycin-resistant Staphylococcus aureus) resistant to vancomycin has been cropping up clinically in recent years, and humans are about to face the situation of no cure.

Studies found that pathogenic factors released by Staphylococcus aureus include hemolysin, leukocidin, enterotoxin, coagulase, and the like. Hemolysin is one of major virulence factors secreted by Staphylococcus aureus and can be divided into four types, alpha-hemolysin, beta-hemolysin, gamma-hemolysin and delta-hemolysin. Alpha-hemolysin (Hla) is a secreted toxin protein encoded by gene HLA of Staphylococcus aureus, expressed in almost all strains. Compared with other types of hemolysin, alpha-hemolysin is a more critical virulence factor affecting pathogenicity of Staphylococcus aureus, and researches showed that Staphylococcus aureus with alpha-hemolysin-expressing HLA gene deleted has a significantly reduced toxicity in infecting animals. Alpha-hemolysin protein has a full length of 319 amino acids and a relative molecular weight of 33 kD, and is secreted in a monomer form. As one of the members of pore forming toxin family, the most well-defined biological property of alpha-hemolysin is the ability to rapidly lyse red blood cells and other tissue cells in host. Alpha-hemolysin binds to cholesterol and sphingomyelin on host cells, and then they aggregate together to form a heptamer with a relative molecular weight of about 232 kD, which in turn folds to form a β-barrel transmembrane structure with a diameter of about 1.5 nm, allowing intracellular water, ions and other small molecules to leak and thereby causing hemolysis or death of target cells. In addition, alpha-hemolysin can also cause the contraction and spasm of smooth muscles of capillary vessels, thereby resulting in the blockage of the capillary vessels and causing ischemia and tissue necrosis. So it plays an important role in the processes that Staphylococcus aureus causes diseases such as sepsis, pneumonia, mammary gland infection, cornea infection, serious skin infection and the like. At the same time, alpha-hemolysin also destroys leukocytes in infected tissues of a host, prevents the host from clearing Staphylococcus aureus infected. Under the pressure of host immune system and antibiotics, Staphylococcus aureus at infected areas releases a great amount of alpha-hemolysin, and when the released alpha-hemolysin enters blood, the host immune system will be activated to release excessive inflammatory factors, thereby forming sepsis.

In case of the continuous emergence of superbacteria, antibody drugs have become a powerful tool of the post-antibiotic era. Antibody is one kind of natural protein produced by adaptive immune system, and a human body is administered with antibody drugs through passive immunization, so that the antibody not only can neutralize virulence factors, but also can strengthen the immune response capability of a host to pathogens, and can accelerate the elimination of infected pathogens. Alpha-hemolysin shows its potential as an anti-infection antibody drug target for the treatment of Staphylococcus aureus infection for the following reasons.

Alpha-hemolysin is highly conserved among Staphylococcus aureus strains and is expressed in almost all Staphylococcus aureus strains; alpha-hemolysin has definite biological functions and plays an important role in the process of forming host infection and developing sepsis by Staphylococcus aureus; and no homologous protein of alpha-hemolysin exists in mammalian cells, so an antibody drug can be designed by targeting alpha-hemolysin, with low potential off-target possibility and low toxic and side effects.

Therefore, alpha-hemolysin is an ideal target for an antibody drug against Staphylococcus aureus infection, and an effective neutralization of alpha-hemolysin is beneficial to blocking Staphylococcus aureus infection in a human body, avoiding occurrence of sepsis after serious infection and improving prognosis of a clinically infected patient. Presently, fully human antibody R-301 (Salvecin^{®}) developed by Aridis Pharmaceuticals, Inc. targeting alpha-hemolysin has entered phase III clinical trial, and main indications are severe pneumonia caused by Staphylococcus aureus (including methicillin-resistant Staphylococcus aureus); humanized antibody MEDI4893 developed by Astrazeneca Pharmaceuticals Inc. targeting alpha-hemolysin is under phase II clinical investigation, and is mainly used for prevention and treatment of Staphylococcus aureus (including methicillin-resistant Staphylococcus aureus) pneumonia.

Currently antibiotics such as vancomycin and the like are still the first line drugs for treating Staphylococcus aureus infection, but in view of the continuous increase in drug resistance and the slow development of new antibiotics, there is still a need in the art to develop novel and highly effective antibody drugs against Staphylococcus aureus by directly neutralizing toxins. Due to the hemolytic activity of Staphylococcus aureus alpha hemolysin, preparation strategies of corresponding monoclonal antibodies mainly include: using an alpha hemolysin-based toxoid as an immunogen to prepare a monoclonal antibody by hybridoma technology; screening an in vitro antibody library by using alpha-hemolysin as a target protein; and the like. These methods have difficulty in obtaining an anti-alpha-hemolysin antibody having high affinity and high biological activity for virulent alpha-hemolysin. Regeneron Pharmaceuticals Inc. adopted an attenuated alpha-hemolysin as immunogen, and detected affinities of positive clones through surface plasma resonance, which however had a low screening efficiency and could not efficiently obtain antibodies with high biological activity.

### SUMMARY OF THE PRESENT INVENTION

The technical problem to be solved by the present invention to improve traditional preparation method of anti-alpha-hemolysin antibody molecules, and following strategies are utilized in the present invention: solving the virulence problem of alpha-hemolysin in animals through attenuated toxicity immunization, ensuring the high affinity of an antibody to wild-type virulent alpha-hemolysin through strong toxicity screening, ensuring the biological activity of a monoclonal antibody from the very beginning through erythrocyte lysis inhibition assay, thereby improving the screening efficiency of anti-alpha-hemolysin therapeutic monoclonal antibodies. Further, the technical problem to be solved by the present invention is to provide an anti-alpha-hemolysin antibody molecule, in particular a humanized anti-alpha-hemolysin monoclonal antibody, which has the ability to bind to Staphylococcus aureus alpha hemolysin and inhibit the hemolysin from lysing blood cells and damaging tissue cells, and can be used alone or in combination with existing anti-bacterial agents for the treatment of infection or infection-related diseases caused by alpha-hemolysin or alpha-hemolysin producing microorganisms.

In view of the above problems, the present invention is directed to provide an antibody or a functional fragment thereof, and uses thereof based on the antibody or the functional fragment thereof.

Technical solutions of the present invention are as follows.

In one aspect, the present invention provides a method for preparing an anti-alpha-hemolysin monoclonal antibody, including steps as follows:
(1) immunizing animals with an attenuated alpha-hemolysin or an alpha-hemolysin without virulence;
(2) taking splenocytes and preparing hybridomas;
(3) screening an antibody having high affinity to virulent alpha-hemolysin or wild-type alpha-hemolysin using the same;
(4) selecting an antibody which has an activity of inhibiting erythrocyte lysis caused by virulent alpha-hemolysin or wild-type alpha-hemolysin through an assay of inhibition of erythrocyte lysis .

The assay of inhibition of erythrocyte lysis includes mixing a diluted antibody (or a hybridoma supernatant) with an equal volume of alpha-hemolysin, adding erythrocytes in for incubation, centrifuging and obtaining a supernatant, and detecting the inhibitory activity of the antibody on erythrocyte lysis by alpha-hemolysin according to OD450.

Preferably, in the method for preparing an anti-alpha-hemolysin monoclonal antibody according to the present invention, the virulent alpha-hemolysin or wild-type alpha-hemolysin is Staphylococcus aureus alpha-hemolysin, and preferably has an amino acid sequence as shown in SEQ ID NO: 78; and the attenuated alpha-hemolysin or alpha-hemolysin without virulence is alpha-hemolysin with H35L, and preferably has an amino acid sequence as shown in SEQ ID NO: 80.

Preferably, in the method for preparing an anti-alpha-hemolysin monoclonal antibody according to the present invention, the virulent alpha-hemolysin or wild-type alpha-hemolysin, the attenuated alpha-hemolysin, or the alpha-hemolysin without virulence is independently linked to a purification tag, a carrier protein, and/or an adjuvant molecule; and the linkage includes coupling, cross-linking, conjugation, and/or fusion.

In another aspect, the present invention also provides an anti-alpha-hemolysin monoclonal antibody prepared by any one of the previous methods, the antibody having at least one activity selected from the group consisting of blocking hemolytic effect of alpha-hemolysin, blocking damage of alpha-hemolysin to lung epithelial cells, reducing bacterial load in MRSA infected lung tissue, prolonging survival time of a patient suffering from MRSA bacteremia, and preventing or alleviating sepsis caused by alpha-hemolysin.

The anti-alpha-hemolysin monoclonal antibody prepared by any one of the previous methods according to the present invention has the following structures:
VH-CDR1: selected from the group consisting of SEQ ID NOs: 1, 2, 3, 12, 13, 14, 22, 23, 24, 34, 35, and 36;
VH-CDR2: selected from the group consisting of SEQ ID NOs: 4, 5, 6, 15, 16, 17, 25, 26, 27, 28, 37, 38, and 39;
VH-CDR3: selected from the group consisting of SEQ ID NOs: 7, 18, 29, and 40;
VL-CDR1: selected from the group consisting of SEQ ID NOs: 8, 19, 30, 31, and 41;
VL-CDR2: selected from the group consisting of SEQ ID NOs: 9, 11, 20, 32, and 42; and
VL-CDR3: selected from the group consisting of SEQ ID NOs: 10, 21, 33, and 43.

In a further aspect, the present invention provides an antibody or fragment thereof comprising a heavy chain variable region (VH) and a light chain variable region (VL), wherein the heavy chain variable region (VH) and the light chain variable region (VL) comprise a combination of CDRs (VH-CDR1, VH-CDR2, VH-CDR3; and VL-CDR1, VL-CDR2, VL-CDR3) selected from the group consisting of:
(1) VH-CDR1 as shown in SEQ ID NO: 1, VH-CDR2 as shown in SEQ ID NO: 4, and VH-CDR3 as shown in SEQ ID NO: 7; and, VL-CDR1 as shown in SEQ ID NO: 8, VL-CDR2 as shown in SEQ ID NO: 9, and VL-CDR3 as shown in SEQ ID NO: 10;
(2) VH-CDR1 as shown in SEQ ID NO: 2, VH-CDR2 as shown in SEQ ID NO: 5, and VH-CDR3 as shown in SEQ ID NO: 7; and, VL-CDR1 as shown in SEQ ID NO: 8, VL-CDR2 as shown in SEQ ID NO: 9, and VL-CDR3 as shown in SEQ ID NO: 10;
(3) VH-CDR1 as shown in SEQ ID NO: 3, VH-CDR2 as shown in SEQ ID NO: 6, and VH-CDR3 as shown in SEQ ID NO: 7; and, VL-CDR1 as shown in SEQ ID NO: 8, VL-CDR2 as shown in SEQ ID NO: 9, and VL-CDR3 as shown in SEQ ID NO: 10;
(4) VH-CDR1 as shown in SEQ ID NO: 2, VH-CDR2 as shown in SEQ ID NO: 6, and VH-CDR3 as shown in SEQ ID NO: 7; and, VL-CDR1 as shown in SEQ ID NO: 8, VL-CDR2 as shown in SEQ ID NO: 9, and VL-CDR3 as shown in SEQ ID NO: 10;
(5) VH-CDR1 as shown in SEQ ID NO: 2, VH-CDR2 as shown in SEQ ID NO: 6, and VH-CDR3 as shown in SEQ ID NO: 7; and, VL-CDR1 as shown in SEQ ID NO: 8, VL-CDR2 as shown in SEQ ID NO: 11, and VL-CDR3 as shown in SEQ ID NO: 10;
(6) VH-CDR1 as shown in SEQ ID NO: 12, VH-CDR2 as shown in SEQ ID NO: 15, and VH-CDR3 as shown in SEQ ID NO: 18; and, VL-CDR1 as shown in SEQ ID NO: 19, VL-CDR2 as shown in SEQ ID NO: 20, and VL-CDR3 as shown in SEQ ID NO: 21;
(7) VH-CDR1 as shown in SEQ ID NO: 13, VH-CDR2 as shown in SEQ ID NO: 16, and VH-CDR3 as shown in SEQ ID NO: 18; and, VL-CDR1 as shown in SEQ ID NO: 19, VL-CDR2 as shown in SEQ ID NO: 20, and VL-CDR3 as shown in SEQ ID NO: 21;
(8) VH-CDR1 as shown in SEQ ID NO: 14, VH-CDR2 as shown in SEQ ID NO: 17, and VH-CDR3 as shown in SEQ ID NO: 18; and, VL-CDR1 as shown in SEQ ID NO: 19, VL-CDR2 as shown in SEQ ID NO: 20, and VL-CDR3 as shown in SEQ ID NO: 21;
(9) VH-CDR1 as shown in SEQ ID NO: 13, VH-CDR2 as shown in SEQ ID NO: 17, and VH-CDR3 as shown in SEQ ID NO: 18; and, VL-CDR1 as shown in SEQ ID NO: 19, VL-CDR2 as shown in SEQ ID NO: 20, and VL-CDR3 as shown in SEQ ID NO: 21;
(10) VH-CDR1 as shown in SEQ ID NO: 22, VH-CDR2 as shown in SEQ ID NO: 25, and VH-CDR3 as shown in SEQ ID NO: 29; and, VL-CDR1 as shown in SEQ ID NO: 30, VL-CDR2 as shown in SEQ ID NO: 32, and VL-CDR3 as shown in SEQ ID NO: 33;
(11) VH-CDR1 as shown in SEQ ID NO: 23, VH-CDR2 as shown in SEQ ID NO: 26, and VH-CDR3 as shown in SEQ ID NO: 29; and, VL-CDR1 as shown in SEQ ID NO: 30, VL-CDR2 as shown in SEQ ID NO: 32, and VL-CDR3 as shown in SEQ ID NO: 33;
(12) VH-CDR1 as shown in SEQ ID NO: 24, VH-CDR2 as shown in SEQ ID NO: 27, and VH-CDR3 as shown in SEQ ID NO: 29; and, VL-CDR1 as shown in SEQ ID NO: 30, VL-CDR2 as shown in SEQ ID NO: 32, and VL-CDR3 as shown in SEQ ID NO: 33;
(13) VH-CDR1 as shown in SEQ ID NO: 23, VH-CDR2 as shown in SEQ ID NO: 27, and VH-CDR3 as shown in SEQ ID NO: 29; and, VL-CDR1 as shown in SEQ ID NO: 30, VL-CDR2 as shown in SEQ ID NO: 32, and VL-CDR3 as shown in SEQ ID NO: 33;
(14) VH-CDR1 as shown in SEQ ID NO: 23, VH-CDR2 as shown in SEQ ID NO: 28, and VH-CDR3 as shown in SEQ ID NO: 29; and, VL-CDR1 as shown in SEQ ID NO: 31, VL-CDR2 as shown in SEQ ID NO: 32, and VL-CDR3 as shown in SEQ ID NO: 33;
(15) VH-CDR1 as shown in SEQ ID NO: 34, VH-CDR2 as shown in SEQ ID NO: 37, and VH-CDR3 as shown in SEQ ID NO: 40; and, VL-CDR1 as shown in SEQ ID NO: 41, VL-CDR2 as shown in SEQ ID NO: 42, and VL-CDR3 as shown in SEQ ID NO: 43;
(16) VH-CDR1 as shown in SEQ ID NO: 35, VH-CDR2 as shown in SEQ ID NO: 38, and VH-CDR3 as shown in SEQ ID NO: 40; and, VL-CDR1 as shown in SEQ ID NO: 41, VL-CDR2 as shown in SEQ ID NO: 42, and VL-CDR3 as shown in SEQ ID NO: 43;
(17) VH-CDR1 as shown in SEQ ID NO: 36, VH-CDR2 as shown in SEQ ID NO: 39, and VH-CDR3 as shown in SEQ ID NO: 40; and, VL-CDR1 as shown in SEQ ID NO: 41, VL-CDR2 as shown in SEQ ID NO: 42, and VL-CDR3 as shown in SEQ ID NO: 43; and
(18) VH-CDR1 as shown in SEQ ID NO: 35, VH-CDR2 as shown in SEQ ID NO: 39, and VH-CDR3 as shown in SEQ ID NO: 40; and, VL-CDR1 as shown in SEQ ID NO: 41, VL-CDR2 as shown in SEQ ID NO: 42, and VL-CDR3 as shown in SEQ ID NO: 43.

Preferably, in the antibody or fragment thereof provided by the present invention, the heavy chain variable region comprises:
an amino acid sequence as shown in any one of SEQ ID NO: 44, SEQ ID NO: 48, SEQ ID NO: 52, SEQ ID NO: 56, SEQ ID NO: 60, SEQ ID NO: 64, SEQ ID NO: 68 and SEQ ID NO: 72, or an amino acid sequence having at least 75% identity to the amino acid sequence as shown; and/or
the light chain variable region comprises:
   an amino acid sequence as shown in any one of SEQ ID NO: 46, SEQ ID NO: 50, SEQ ID NO: 54, SEQ ID NO: 58, SEQ ID NO: 62, SEQ ID NO: 66, SEQ ID NO: 70, SEQ ID NO: 74 and SEQ ID NO: 76 or an amino acid sequence having at least 75% identity to the amino acid sequence as shown.

According to a particular embodiment of the present invention, the antibody or fragment thereof comprises a heavy chain variable region and a light chain variable region selected from the group consisting of following combinations:
(1) an amino acid sequence as shown in SEQ ID NO: 44 or an amino acid sequence having at least 75% identity to the amino acid sequence as shown in SEQ ID NO: 44; and, an amino acid sequence as shown in SEQ ID NO: 46 or an amino acid sequence having at least 75% identity to the amino acid sequence as shown in SEQ ID NO: 46;
(2) an amino acid sequence as shown in SEQ ID NO: 48 or an amino acid sequence having at least 75% identity to the amino acid sequence as shown in SEQ ID NO: 48; and, an amino acid sequence as shown in SEQ ID NO: 50 or an amino acid sequence having at least 75% identity to the amino acid sequence as shown in SEQ ID NO: 50;
(3) an amino acid sequence as shown in SEQ ID NO: 52 or an amino acid sequence having at least 75% identity to the amino acid sequence as shown in SEQ ID NO: 52; and, an amino acid sequence as shown in SEQ ID NO: 54 or an amino acid sequence having at least 75% identity to the amino acid sequence as shown in SEQ ID NO: 54;
(4) an amino acid sequence as shown in SEQ ID NO: 56 or an amino acid sequence having at least 75% identity to the amino acid sequence as shown in SEQ ID NO: 56; and, an amino acid sequence as shown in SEQ ID NO: 58 or an amino acid sequence having at least 75% identity to the amino acid sequence as shown in SEQ ID NO: 58;
(5) an amino acid sequence as shown in SEQ ID NO: 60 or an amino acid sequence having at least 75% identity to the amino acid sequence as shown in SEQ ID NO: 60; and, an amino acid sequence as shown in SEQ ID NO: 62 or an amino acid sequence having at least 75% identity to the amino acid sequence as shown in SEQ ID NO: 62;
(6) an amino acid sequence as shown in SEQ ID NO: 64 or an amino acid sequence having at least 75% identity to the amino acid sequence as shown in SEQ ID NO: 64; and, an amino acid sequence as shown in SEQ ID NO: 66 or an amino acid sequence having at least 75% identity to the amino acid sequence as shown in SEQ ID NO: 66;
(7) an amino acid sequence as shown in SEQ ID NO: 68 or an amino acid sequence having at least 75% identity to the amino acid sequence as shown in SEQ ID NO: 68; and, an amino acid sequence as shown in SEQ ID NO: 70 or an amino acid sequence having at least 75% identity to the amino acid sequence as shown in SEQ ID NO: 70;
(8) an amino acid sequence as shown in SEQ ID NO: 72 or an amino acid sequence having at least 75% identity to the amino acid sequence as shown in SEQ ID NO: 72; and, an amino acid sequence as shown in SEQ ID NO: 74 or an amino acid sequence having at least 75% identity to the amino acid sequence as shown in SEQ ID NO: 74; and
(9) an amino acid sequence as shown in SEQ ID NO: 48 or an amino acid sequence having at least 75% identity to the amino acid sequence as shown in SEQ ID NO: 48; and, an amino acid sequence as shown in SEQ ID NO: 76 or an amino acid sequence having at least 75% identity to the amino acid sequence as shown in SEQ ID NO: 76.

The at least 75% identity described above is any percent identity greater than or equal to 75%, such as at least 80%, preferably at least 85%, more preferably at least 90%, even more preferably at least 91%, 92%, 93%, 94%, 95%, 96%, 97%, 98%, or even 99% identity.

The antibody or fragment thereof provided by the present invention can be in any form, for example, a monoclonal antibody, a single-chain antibody, a single-domain antibody, a bifunctional antibody, a nanobody, a fully or partially humanized antibody or a chimeric antibody and the like; alternatively, the antibody or fragment thereof is a half-antibody or an antigen-binding fragment of the half-antibody, for example, single-chain variable fragment (scFv), bivalent single-chain variable fragment (BsFv), disulfide-stabilized Fv fragment (dsFv), (disulfide-stabilized Fv fragment)₂ (dsFv)₂, antigen-binding fragment (Fab), Fab' fragment, F(ab')₂ fragment, or variable fragment (Fv); and with respect to the fragment of the antibody provided by the present invention, preferably, the fragment is any fragment of the antibody capable of specifically binding to the antigen Staphylococcus aureus alpha-hemolysin.

Alternatively, the antibody according to the present invention is IgA, IgD, IgE, IgG or IgM, more preferably IgG1. An fragment of the antibody is selected from the group consisting of scFv, Fab, F(ab')₂ and Fv fragments of the antibody.

Preferably, the antibody or fragment thereof further comprises a human or murine constant region, preferably a human or murine light chain constant region (CL) and/or heavy chain constant region (CH); more preferably, the antibody or fragment thereof comprises a heavy chain constant region selected from the group consisting of IgG, IgA, IgM, IgD and IgE and/or a kappa or lambda type light chain constant region. According to a particular embodiment of the present invention, the antibody is a monoclonal antibody, preferably murine derived, chimeric or humanized monoclonal antibody; more preferably, the heavy chain constant region of the monoclonal antibody is of IgG1 or IgG4 subtype and the light chain constant region of the monoclonal antibody is of kappa type.

Preferably, the antibody or fragment thereof provided by the present invention comprises a heavy chain constant region as shown in SEQ ID NO: 86 and/or a light chain constant region as shown in SEQ ID NO: 87, or an amino acid sequence having at least 75% identity to either the heavy chain constant region or the light chain constant region as shown.

In still a further aspect, the present invention provides a nucleic acid molecule comprising a nucleotide sequence encoding any antibody or fragment thereof of the present invention, or encoding a heavy chain CDR, a light chain CDR, a heavy chain variable region, a light chain variable region, a heavy chain or a light chain comprised in the antibody or fragment thereof.

According to a particular embodiment of the present invention, the nucleic acid molecule comprises a nucleotide sequence encoding the heavy chain variable region or the light chain variable region of the antibody or fragment thereof according to the present invention. For example, the nucleic acid molecule comprises the nucleotide sequence as shown in any one of SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, and SEQ ID NO: 77.

In yet another aspect, the present invention provides a vector comprising the nucleic acid molecule according to the present invention. The vector can be an eukaryotic expression vector, a prokaryotic expression vector, an artificial chromosome, a phage vector, and the like.

The vector or nucleic acid molecule of the present invention may be used to transform or transfect a host cell or in any way enter a host cell for antibody preservation or expression, etc. Thus, in a further aspect, the present invention provides a host cell comprising the nucleic acid molecule and/or vector according to the present invention, or transformed or transfected with the nucleic acid molecule and/or vector according to the present invention. The host cell may be any prokaryotic or eukaryotic cell, such as a bacterial or insect, fungus, plant or animal cell.

According to the disclosure of the present invention, the antibody or fragment thereof, the nucleic acid molecule, the vector, and/or the host cell provided by the present invention can be obtained using any conventional techniques known in the art. For example, with respect to an antibody, the heavy chain variable region and/or the light chain variable region of the antibody or the heavy chain and/or the light chain of the antibody may be obtained from the nucleic acid molecule provided by the present invention, and then the antibody is obtained by assembling them with optional other domains of the antibody; alternatively, the host cell provided by the present invention is cultured under conditions that allow the host cell to express the heavy chain variable region and/or the light chain variable region of the antibody or the heavy chain and/or the light chain of the antibody and assemble them into an antibody. Optionally, the method further includes a step of recovering the produced antibody.

The antibody or fragment thereof provided by the present invention may also be combined with other moieties, for example, a cell surface receptor, a small molecule compound such as amino acids and carbohydrates, a small molecule polymer, or any other moiety that modifies the antibody of the present invention, or even an active protein or polypeptide such as antimicrobial peptides or antibiotics. Thus, in another aspect, the present invention provides a conjugate or fusion protein comprising an antibody or fragment thereof provided by the present invention. For example, the conjugate or fusion protein can be a bispecific antibody comprising an antibody or fragment thereof according to the present invention.

The antibody or fragment thereof, the nucleic acid molecule, the vector, the host cell, the conjugate or fusion protein provided by the present invention may be contained in a pharmaceutical composition, more particularly, a pharmaceutical preparation, to be used for various purposes as actually needed. Thus, in a further aspect, the present invention also provides a pharmaceutical composition comprising an antibody or fragment thereof, a nucleic acid molecule, a vector, a host cell, a conjugate and/or a fusion protein according to the present invention, and optionally a pharmaceutically acceptable excipient.

For any purpose of use, the present invention also provides a kit comprising an antibody or fragment thereof, a nucleic acid molecule, a vector, a host cell, a conjugate, a fusion protein and/or a pharmaceutical composition of the present invention.

Based on their ability to bind to alpha-hemolysin and inhibit the hemolysin from lysing blood cells and damaging tissue cells, the antibody or fragment thereof according to the present invention can be used alone or in combination with any other antibacterial agent, to treat or ameliorate infection caused by alpha-hemolysin or alpha-hemolysin producing microorganisms, or other diseases or conditions resulting from the infection. Accordingly, the present invention also provides related applications of the subject matters described above.

In particular, in still a further aspect, the present invention provides use of an antibody or fragment thereof, a nucleic acid molecule, a vector, a host cell, a conjugate, a fusion protein and/or a pharmaceutical composition according to the present invention in the manufacture of a medicament for preventing or treating infection and complication caused by alpha-hemolysin or an alpha-hemolysin producing microorganism.

Furthermore, the present invention provides use of the antibody or fragment thereof, the nucleic acid molecule, the vectors, the host cell, the conjugate, the fusion protein and/or the pharmaceutical composition in combination with another antibacterial agent or anti-alpha-hemolysin antibody in the manufacture of a medicament for preventing or treating infection and complication caused by alpha-hemolysin or an alpha-hemolysin producing microorganism.

In addition, the present invention provides a method for preventing or treating infection and complication caused by alpha-hemolysin or an alpha-hemolysin producing microorganism, including administering to a subject in need thereof the antibody or fragment thereof, the nucleic acid molecule, the vector, the host cell, the conjugate, the fusion protein and/or the pharmaceutical composition, and optionally an antibacterial agent. The optional antibacterial agent may be an agent co-administered with the antibody or fragment thereof, the nucleic acid molecule, the vector, the host cell, the conjugate, the fusion protein and/or the pharmaceutical composition of the present invention. The co-administration of the two may be in any way, including simultaneously, sequentially or at intervals.

In the present invention, the alpha-hemolysin producing microorganism is preferably Staphylococcus aureus, including methicillin-resistant Staphylococcus aureus.

In the present invention, the infection caused by alpha-hemolysin or an alpha-hemolysin producing microorganism may be one or more selected from the group consisting of upper respiratory tract infection, pneumonia, severe pneumonia, abdominal infection, subcutaneous and soft tissue infection, bacteremia, and infection in various organs; and the complication may be one or more selected from the group consisting of Acute Respiratory Distress Syndrome (ARDS), sepsis, and a rise in inflammatory factors in body.

In the present invention, the another antibacterial agent is an agent (including chemical agents, biological agents and Chinese medicines) useful for the treatment and prevention of infection caused by Staphylococcus aureus, e.g. methicillin-resistant Staphylococcus aureus, preferably is an antibiotic, such as beta-lactam antibiotics. The antibiotic may be a drug listed in guidelines/treatment strategies issued by the Infectious Diseases Society of America (IDSA) or the Chinese Medical Association, preferably vancomycin, norvancomycin, teicoplanin, linezolid, daptomycin, cephapiprazole, fusidic acid, or ceftaroline.

The present invention also provides a method for diagnosing infection caused by alpha-hemolysin or an alpha-hemolysin producing microorganism, including contacting the antibody or fragment thereof, the nucleic acid molecule, the vector, the host cell, the conjugate, the fusion protein and/or the pharmaceutical composition with a sample from a subject.

In the present invention, a "subject" may be a mammal, such as a primate or rodent, more preferably a human.

In the present invention, terms "alpha-hemolysin" and "alpha-hemolysin toxin" interchangeably refer to a 33 kDa monomeric protein secreted by Staphylococcus aureus that oligomerizes into a heptameric structure in host cell membrane to form pores, resulting in cell lysis, inflammation, and tissue damage. The amino acid sequence of wild-type alpha-hemolysin is as shown in SEQ ID NO: 78. The amino acid sequence of modified alpha-hemolysin (H35L mutant) is as shown in SEQ ID NO: 80. Unless otherwise indicated, reference to alpha-hemolysin refers to the wild-type form. Hla-H35L is an alpha-hemolysin with histidine at position 35 changed to leucine. This allows the toxin to assemble on the host cell membrane till the pre-pore forms, but does not form a fully functional pore. This renders the alpha-hemolysin non-toxic.

In the present invention, term "antibody" is intended to refer to an immunoglobulin molecule comprised of four polypeptide chains, two heavy (H) chains and two light (L) chains interconnected by disulfide bonds (i.e., "full antibody molecule"), as well as a multimer thereof (e.g., IgM) or an antigen-binding fragment thereof. Each heavy chain comprises a heavy chain variable region ("HCVR" or "VH") and a heavy chain constant region (comprising domains CH1, CH2 and CH3). Each light chain comprises a light chain variable region ("LCVR" or "VL") and a light chain constant region (CL). The VH and VL regions can be further subdivided into regions of hypervariability, termed complementarity determining regions (CDRs), interspersed with regions that are more conserved, termed framework regions (FRs). Each VH and VL is composed of three CDRs and four FRs, arranged from amino-terminus to carboxy-terminus in the following order: FR1, CDR1, FR2, CDR2, FR3, CDR3, FR4. In certain embodiments of the present invention, the FRs of the antibody (or antigen binding fragment thereof) may be identical to human germline sequences, or may be naturally or artificially modified. An amino acid consensus sequence may be defined based on a side-by-side analysis of two or more CDRs.

Substitution of one or more CDR residues or omission of one or more CDRs is also possible. Antibodies have been described in scientific literatures in which one or two CDRs can be dispensed with for binding. Padlan et al. (1995 FASEB J. 9: 133-139) analyzed the contact regions between antibodies and their antigens, based on published crystal structures, and concluded that only about one fifth to one third of CDR residues actually contact the antigen. Padlan also found many antibodies in which one or two CDRs had no amino acids in contact with an antigen (see also, Vajdos et al. 2002 J Mol Biol 320: 415-428). CDR residues not contacting antigen can be identified based on previous studies (for example residues H60-H65 in CDRH2 are often not required), from regions of Kabat CDRs lying outside Chothia CDRs, by molecular modeling and/or empirically. If a CDR or residue(s) thereof is omitted, it is usually substituted with an amino acid occupying the corresponding position in another human antibody sequence or a consensus of such sequences. Positions for substitution within CDRs and amino acids to substitute can also be selected empirically. Empirical substitutions can be conservative or non-conservative substitutions.

Term "specifically binds" or "binds specifically to", or the like, means that an antibody or antigen-binding fragment thereof forms a complex with an antigen that is relatively stable under physiologic conditions. Specific binding can be characterized by an equilibrium dissociation constant of at least about 1×10⁻⁶ M or less (e.g., a smaller KD denotes a tighter binding). Methods for determining whether two molecules specifically bind are well known in the art and include, for example, equilibrium dialysis, surface plasmon resonance, and the like. As described herein, antibodies have been identified by surface plasmon resonance, e.g., BIACORE^{™}, to bind specifically to alpha-hemolysin. Moreover, multi-specific antibodies that bind to one domain in alpha-hemolysin and one or more additional antigens or a bi-specific antibodies that bind to two different regions of alpha-hemolysin are nonetheless considered antibodies that "specifically bind", as used herein.

Term "high affinity" antibody refers to those mAbs having a binding affinity to alpha-hemolysin, expressed as KD, of at least 10⁻⁷ M, preferably 10⁻⁸ M, more preferably 10⁻⁹ M, even more preferably 10⁻¹⁰ M, even more preferably 10⁻¹¹ M, as measured by surface plasmon resonance, e.g., BIACORE^{™} or solution-affinity ELISA.

Terms "slow off rate", "K dissociation" and "kd" are intended to refer to an antibody that dissociates from alpha-hemolysin with a rate constant of 1×10⁻³ s⁻¹ or less, preferably 1×10⁻⁴ s⁻¹ or less, as determined by surface plasmon resonance, e.g., BIACORE^{™}.

Terms "antigen-binding portion", "antigen-binding fragment" of an antibody, and the like, as used herein, include any naturally occurring, enzymatically obtainable, synthetic, or genetically engineered polypeptide or glycoprotein that specifically binds an antigen to form a complex. Term "antigen-binding fragment" of an antibody or "antibody fragment", as used herein, refers to one or more fragments of an antibody that retains the ability to bind to alpha-hemolysin.

In particular embodiments, the antibody or antibody fragment of the present invention may be conjugated to a therapeutic moiety ("immunoconjugate"), for example an antibiotic, a second anti-alpha-hemolysin antibody or an antibody directed against a cytokine such as IL-1, IL-6 or TGF-β or any other therapeutic moiety suitable for use in the treatment of a disease or condition including Staphylococcus aureus infection, skin and soft tissue infection (including abscess), surgical site infection, artificial joint infection, bacteremia, sepsis, septic arthritis, meningitis, osteomyelitis, endocarditis, pneumonia, toxic shock syndrome, mastitis and furuncle, and carbuncle (boil).

"Isolated antibody" as used herein, is intended to refer to an antibody that is substantially free of other antibodies (Abs) having different antigenic specificities (e.g., an isolated antibody that specifically binds alpha-hemolysin, or a fragment thereof, is substantially free of Abs that specifically bind antigens other than alpha-hemolysin).

Term "KD", as used herein, is intended to refer to the equilibrium dissociation constant of a particular antibody-antigen interaction.

Compared with prior arts, Staphylococcus aureus alpha-hemolysin (alpha-Toxin) and a Staphylococcus aureus alpha-hemolysin mutant protein (alpha-Toxin H35L) without toxicity were obtained in the present invention by prokaryotic expression in Escherichia coli. Mice were successfully immunized with alpha-Toxin H35L and spleen cells were taken, and an antibody library was established using hybridoma technology. Anti-alpha-hemolysin monoclonal antibodies having high affinity to alpha-hemolysin (alpha-Toxin) and biological activity were obtained through library screening, and lead antibody molecules (of IgG1 subtype) were obtained after the anti-alpha-hemolysin monoclonal antibodies were further humanized.

Specifically, in the present invention mice were successfully immunized with alpha-Toxin H35L and spleen cells were taken, and an antibody library was established using hybridoma technology; anti-alpha-hemolysin monoclonal antibodies having high affinity to alpha-hemolysin (alpha-Toxin) and biological activity were obtained through library screening, and 16 lead antibody molecules in total were obtained, which not only have high affinity to alpha-hemolysin, but also have the activity of blocking hemolysis caused by alpha-hemolysin. Particularly, attenuated toxicity immunization and strong toxicity screening were adopted in the present invention with respect to antigen selection and antibody screening.

Human-mouse chimeric antibodies and humanized antibodies were recombinantly expressed based on the murine-derived lead antibody molecules, and finally humanized antibody molecules with biological activities similar to those of the murine-derived antibodies were obtained.

Pharmacodynamic studies in vitro showed the humanized antibodies of the present invention could, in a dose-dependent manner, block the hemolytic effect of alpha-hemolysin on rabbit erythrocytes and the damage effect of alpha-hemolysin on lung epithelial cells. In addition, in the present invention, an alpha-hemolysin-induced sepsis model, an MRSA bacteremia model and an MRSA lung infection model established in mice were utilized to carry out pharmacodynamic evaluation on the lead antibody molecules in animals. Research results showed that the humanized antibodies of the present invention had a remarkable protective effect on mice in the alpha-hemolysin-induced sepsis model; were capable of remarkably prolonging the survival time of mice in the MRSA bacteremia model; and were able to obviously reduce the bacterial load of tissues in mice in MRSA lung infection model. Moreover, the combined application of the humanized antibodies of the present application and conventional antibacterial agents such as vancomycin and linezolid, etc., showed a remarkable synergistic effect in the alpha-hemolysin-induced sepsis model, the MRSA bacteremia model and the MRSA lung infection model established in mice.

Furthermore, a pharmacokinetic study of a single dose of an antibody of the present invention was performed in cynomolgus monkeys at a dosage of 20 mg/kg (N = 3), and the results showed that basic pharmacokinetic parameters met druggability criteria (Table 1): the in vivo elimination half-life (T1/2) was 137 ± 36.9 h, and the plasma clearance (CL) was 0.501 ± 0.222 ml/h/kg. In addition, an acute toxicity test research of an antibody of the present invention was carried out in mice (N = 10), and the result showed that when a dosage of 125 mg/kg at the maximum was administered in 24 hours, no mice died, and no discomfort was shown by the animals during continuous observation for 14 days; no abnormality was seen in main organs (heart, liver, spleen, lung, kidney and brain) taken for histology observation from sacrificed animals; and when cynomolgus monkeys were administrated with a dosage of 10 mg/kg of an antibody of the present invention once, no death of the animals occurred, and no discomfort was shown by the animals during continuous observation for 28 days. Acute toxicity test research showed that the antibody of the present invention had good safety.

The antibody according to the present invention can effectively neutralize toxin, block damage of the toxin to tissue cells of a patient, and improve immunity of the patient, thereby relieving tissue injury due to clinical Staphylococcus aureus infection, promoting the infected bacteria in the body of the patient to be cleared more quickly, and preventing or relieving sepsis. Under the action of the antibody, patients can be more quickly converted from intravenous infusion treatment to oral treatment, and the treatment course can be shortened; meanwhile, the antibody according to the present invention has better clinical curative effect and tolerance, and is a favorable supplement for the existing antibiotic therapy.

### BRIEF DESCRIPTION OF THE DRAWINGS

Embodiments of the present invention are described in detail below with reference to the attached drawing figures, in which:
FIG. 1 shows the construction of a recombinant expression plasmid for His-tag fused Staphylococcus aureus alpha-hemolysin (alpha-Toxin) protein.
FIG. 2 shows the construction of a recombinant expression plasmid for His-tag fused Staphylococcus aureus alpha-hemolysin mutant (H35L alpha-Toxin) protein.
FIG. 3 shows the SDS-PAGE electrophoresis (10% gels) results of recombinantly expressed Staphylococcus aureus alpha-hemolysin and its mutant (H35L alpha-Toxin), in which panel 3A shows alpha-Toxin, and panel 3B shows H35L alpha-Toxin, and the loading amounts are 10 µg.
FIG. 4 shows the hemolytic effect of Staphylococcus aureus alpha-hemolysin and its mutant (H35L alpha-Toxin) on sheep blood agar plates.
FIG. 5 shows the hemolytic effect of Staphylococcus aureus alpha-hemolysin and its mutant (H35L alpha-Toxin) on rabbit blood, in which panel 5A shows alpha-Toxin and panel 5B shows H35L alpha-Toxin.

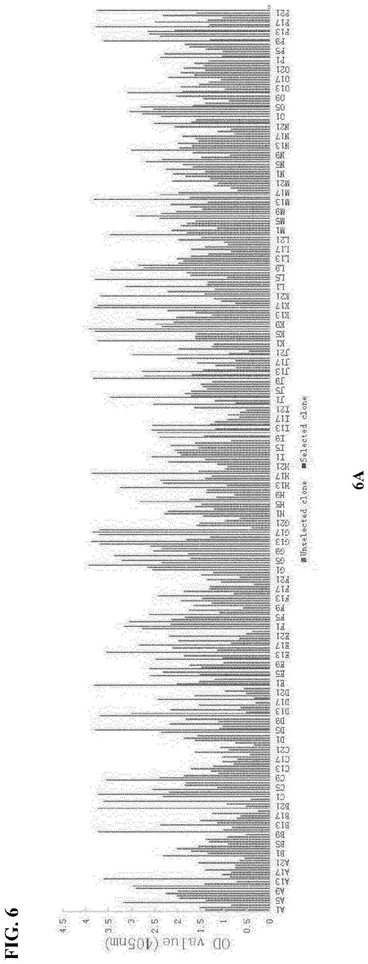

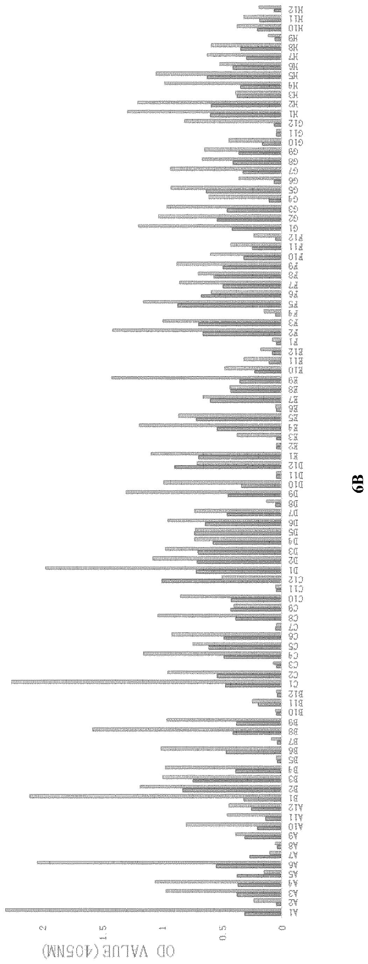

FIG. 6 shows the detection results obtained during screening of hybridoma cell strains, in which panel 6A shows the ELISA detection results of the binding of antibodies in the supernatants of different cell strains to alpha-hemolysin, and panel 6B shows the inhibition results of antibodies in the supernatants of different cell strains on alpha-hemolysin, respectively.
FIG. 7 shows the ELISA detection results of the binding of antibodies of the present invention to alpha-hemolysin, in which panels 7A to 7D show the binding of the screened antibodies 78D4, 16H4, 78F4 and 98G9 to alpha-hemolysin, respectively.
FIG. 8 shows the curves of association and dissociation as detected by Octct of antibodies of the present invention and alpha-hemolysin, in which panels 8A to 8D show the binding of the screened humanized versions of antibodies 78D4, 16H4, 78F4 and 98G9 to alpha-hemolysin, respectively.
FIG. 9 shows the effects of antibodies of the present invention on the hemolytic activity of alpha-hemolysin, in which panels 9A to 9C show the results at different antibody amounts, respectively.
FIG. 10 shows the therapeutic effects of antibodies of the present invention in animal model of alpha-hemolysin-induced sepsis.
FIG. 11 shows the therapeutic effects of antibodies of the present invention in animal model of methicillin-resistant Staphylococcus aureus-induced bacteremia.
FIG. 12 shows the therapeutic effects of antibodies of the present invention in animal model of methicillin-resistant Staphylococcus aureus pneumonia.
Fig. 13 shows the results of a pharmacokinetic study of antibody of the present invention after a single administration in cynomolgus monkeys.

### DETAILED DESCRIPTION OF PREFERRED EMBODIMENTS

The present invention is illustrated below with reference to specific examples. It will be understood by those skilled in the art that these examples are merely illustrative of the present invention and do not limit the scope of the present invention in any way.

Experimental procedures in the following examples are all conventional ones, unless otherwise specified. Raw materials and reagents used in the following examples are all commercially available products, unless otherwise specified.

Known antibodies as follows are used herein as controls:
Fully human antibody R-301 (Salvecin^{®}), abbreviated as AR (see US9249215B2), from Aridis Pharmaceuticals, Inc., of which heavy chain variable region is as shown in SEQ ID NO: 82 and light chain variable region is as shown in SEQ ID NO: 83.

Humanized antibody MEDI4893, abbreviated as AZ (see US20140072577A1), from Astrazeneca Pharmaceuticals Inc., of which heavy chain variable region is as shown in SEQ ID NO: 84 and light chain variable region is as shown in SEQ ID NO: 85.

The antibodies provided by the present invention as follows have a heavy chain constant region as shown in SEQ ID NO: 86 and a light chain constant region as shown in SEQ ID NO: 87.

### Example 1 Recombinant expression of His-tag fused Staphylococcus aureus alpha-hemolysin (alpha-Toxin)

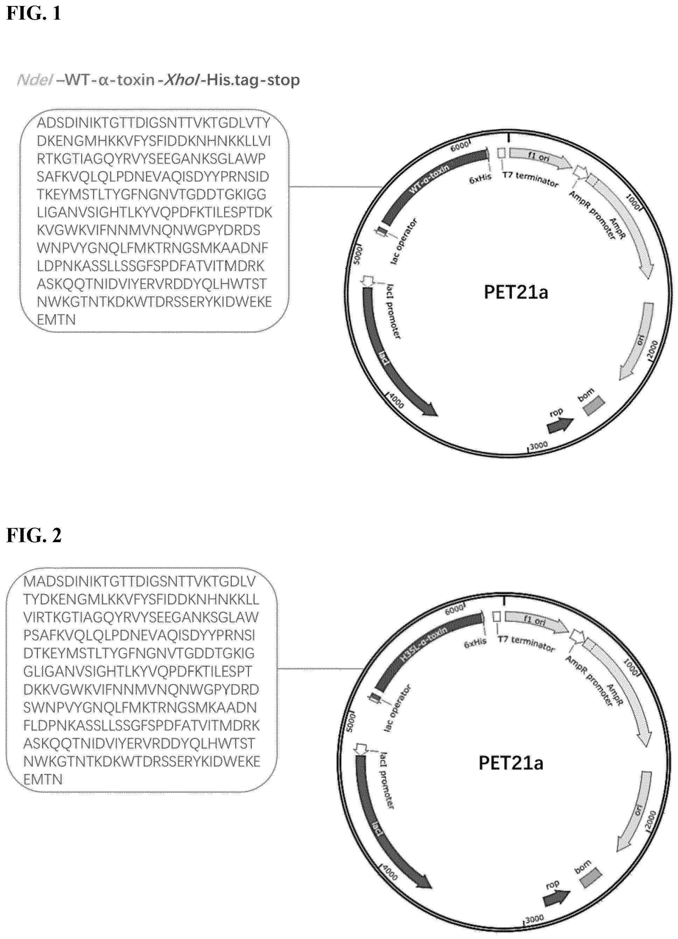

A nucleotide sequence corresponding to the amino acid sequence of Staphylococcus aureus alpha-hemolysin which was used as a target sequence was artificially synthesized, and was cloned into plasmid pET-21a containing His tag utilizing sites for restriction enzymes NdeI and XhoI. The amino acid sequence of Staphylococcus aureus alpha-hemolysin is as shown in SEQ ID NO: 78, the corresponding nucleotide sequence is as shown in SEQ ID NO: 79, and the construction of the recombinant plasmid is shown in FIG. 1.

The obtained recombinant plasmid was transformed into competent BL21(DE3) pLysS cells, and single colonies were picked up next day and inoculated into LB liquid medium containing 100 µg/mL ampicillin, followed by shaking culture at 37 °C overnight. The overnight culture was inoculated into LB liquid medium containing 100 µg/mL ampicillin in a volume ratio of 1:100, and a shaking culture was performed at 37 °C at 200 rpm till OD₆₀₀ of about 0.6-0.8. IPTG was added into the culture to reach a final concentration of 0.1 mM, and induction was performed at 16 °C for 16-18 h. After induction, the bacterial culture was taken and centrifuged at 8,000 rpm for 3 min, and bacterial cells were collected and preserved at -80 °C.

### Example 2 Recombinant expression of His-tag fused Staphylococcus aureus alpha-hemolysin mutant (H35L alpha-Toxin)

Based on the amino acid sequence of Staphylococcus aureus alpha-hemolysin, a mutated amino acid sequence was obtained by mutating histidine (His) at position 35 to leucine (Leu). A nucleotide sequence corresponding to the mutated amino acid sequence was artificially synthesized, and was cloned into plasmid Pet-21a containing His tag utilizing sites for restriction enzymes NdeI and XhoI. The amino acid sequence of Staphylococcus aureus alpha-hemolysin mutant (H35L alpha-Toxin) is as shown in SEQ ID NO: 80 (H35L is at position 36 in that sequence), the corresponding nucleotide sequence is as shown in SEQ ID NO: 81, and the construction of the recombinant plasmid is shown in FIG. 2.

The obtained recombinant plasmid was transformed into competent BL21(DE3) pLysS cells, and single colonies were picked up next day and inoculated into LB liquid medium containing 100 µg/mL ampicillin, followed by shaking culture at 37 °C overnight. The overnight culture was inoculated into LB liquid medium containing 100 µg/mL ampicillin in a volume ratio of 1:100, and a shaking culture was performed at 37 °C at 200 rpm till OD₆₀₀ of about 0.6-0.8. IPTG was added into the culture to reach a final concentration of 0.25 mM, and induction was performed at 25 °C for 4.5 h. After induction, the bacterial culture was taken and centrifuged at 8,000 rpm for 3 min, and bacterial cells were collected and preserved at -80 °C.

### Example 3 Purification of Staphylococcus aureus alpha-hemolysin (alpha-Toxin) and its mutant (H35L alpha-Toxin)

Escherichia coli cells with Staphylococcus aureus alpha-hemolysin and its mutant inducibly expressed therein were sonicated respectively with an ultrasonic system at 180 W with a mode of operating for 3 sec, and pausing for 3 sec, 7-9 min in total. After centrifugation at 13,000 rpm for 30 min, supernatant was collected and sterilized by filtration through a 0.22 µm filter.

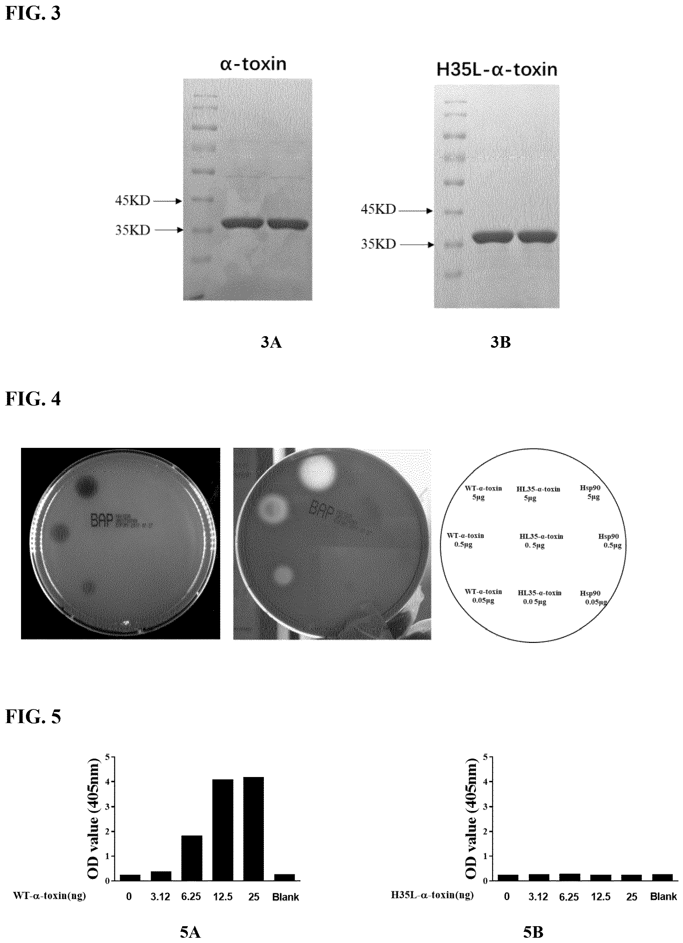

A Ni column was packed with filler and then was mixed with the filtered supernatant on a rotary mixer for 1 h at room temperature. The Ni column was firstly eluted with 5 column volumes of Buffer 1 (containing imidazole at a concentration of 30 mM) till the color of developing solution did not change, to remove proteins non-specifically bound to the column, and then was eluted with 5 column volumes of Buffer 2 (containing imidazole at a concentration of 300 mM) to obtain target proteins. Then, eluates containing target proteins were concentrated and displaced using a 10 KD concentrator in PBS as a solvent. The electrophoresis results of the obtained protein are shown in FIG. 3.

### Example 4 Detection of biological activity of Staphylococcus aureus alpha-hemolysin (alpha-Toxin) and its mutant (H35L alpha-Toxin) (in vitro hemolysis assay and in vivo toxicity assay in mice)

Respectively, 10 µL of each of Staphylococcus aureus alpha-hemolysin and its mutant in different concentrations (5 µg/mL, 0.5 µg/mL and 0.05 µg/mL) was dripped on the surface of a sheep blood agar plate (Shanghai Comagal Microbial Technology CO., LTD), and the plates were placed in an incubator at 37 °C for incubation for 24 h. Then hemolytic rings were obviously visible around the alpha-hemolysin dripped, having diameters related to concentrations of the alpha-hemolysin dripped; while no hemolytic ring was found around the alpha-hemolysin mutant dripped. The results of the assay are shown in FIG. 4.

75 µL of 5% rabbit erythrocytes (Bio-channel Biotechnology) and different amounts of Staphylococcus aureus alpha-hemolysin or its mutant were added to the wells of a 96-well plate, and PBS was added to obtain a final volume of 150 µL. The 96-well plate was incubated in an incubator at 37 °C for 1 h, and then was centrifuged at 3000 rpm for 3 min. 100 µL of the supernatant from each well was evaluated for hemolytic activity by absorbance at 405 nm (OD405) measured using a microplate reader. The results showed that alpha-hemolysin had a dose-dependent hemolytic activity on rabbit erythrocytes, whereas the alpha-hemolysin mutant had no hemolytic activity. The results of the assay are shown in FIG. 5.

Different dosages of Staphylococcus aureus alpha-hemolysin or its mutant recombinantly expressed according to the methods described in Examples 1, 2 and 3 were administered to C57 mice by tail vein injection. It was found that the lowest lethal dosage of Staphylococcus aureus alpha-hemolysin in C57 mice was 3 µg/mouse; while no any discomfort was seen with the mice administered with the highest dosage 200 µg/mouse of the alpha-hemolysin mutant.

### Example 5 Immunization of C57 mice with Staphylococcus aureus alpha-hemolysin mutant (H35L alpha-Toxin)

Alpha-hemolysin or alpha-hemolysin mutant was diluted to different concentrations with PBS and injected via tail vein into C57 mice. Toxic effects of the two on C57 mice were compared, to choose an immunogen and dosage thereof suitable for the subsequent immunization of animals. The results are shown in Table 1.

**Table 1 Toxic effects of different dosages of alpha-hemolysin and alpha-hemolysin mutant on mice**

| **Protein** | **Dosage** | **Performance of the mice** |
|---|---|---|
| **WT α-toxin** | 50 µg/mouse (2.5 mg/kg) | Instantly died |
| | 10 µg/mouse (0.5 mg/kg) | Instantly died |
| | 3 µg/mouse (0.15 mg/kg) | Died within 30 min |
| | 1 µg/mouse (0.05 mg/kg) | Discomforts including tremble, fidget, and the like exhibited; survived; and recovered 16 hours later (overnight) |
| | 0.1 µg/mouse (0.005 mg/kg) | No obvious discomfort shown |
| **H35L α-toxin** | 200 µg/mouse (10 mg/kg) | No obvious discomfort shown |
| **Unrelated protein BSA** | 200 µg/mouse (10 mg/kg) | No obvious discomfort shown |

### Example 6 Immunization of Balb/c mice with Staphylococcus aureus alpha-hemolysin mutant (H35L alpha-Toxin)

8 week old Balb/c mice were immunized using a procedure of 42 days in total at 14 day interval, with reference to Antibodies a Laboratory Manual, Second Edition (Edward A. Greenfield 2012).

The Staphylococcus aureus alpha-hemolysin mutant was emulsified in complete or incomplete Freund's adjuvant and injected into the subcutaneous tissue and peritoneal cavity of the nape of neck, tail root and groin of each mouse in a unilateral manner. On the 35th day of immunization, tail vein blood was collected, antibody titer was detected by ELISA, and spleen cells of the immunized mice were collected and fused with myeloma cells.

### Example 7 Screening and identification of hybridoma cell strains and sequencing of antibodies

Spleen cells of Balb/c mice immunized with Staphylococcus aureus alpha-hemolysin mutant (H35L alpha-Toxin) were taken and fused with myeloma cells P3X63Ag8.653 using PEG or electrofusion method. The fused hybridoma cells were inoculated in 30 384-well plates, and 24 hours later, HAT-containing medium and HT-containing medium were added to select hybridoma cells. After cultured in 384-well plates for 10-14 days, supernatants of cell cultures were taken and subjected to ELISA assay with α-Toxin to screen hybridoma clones capable of secreting antibodies that specifically bind to alpha-hemolysin (see panel 6A). Subsequently, OD values from the wells detected by ELISA were ordered from the highest to the lowest, and wells per plate with values in the top 94 were selected and cells therein were transferred to a 96-well plate for culture (among the 384-well plates, plate NO. 30 was less positive for ELISA detection, so no wells of the plate were selected for transferring); and 29 96-well plates in total were transferred.

Under physiological conditions, the presence of alpha-hemolysin leads to lysis of red blood cells, and the release of lysates leads to a change in the color of the supernatant. An anti-alpha-hemolysin antibody in the supernatant secreted by cells can inhibit alpha-hemolysin from lysing red blood cells. With the detection of absorbance of the supernatant, the degree of lysis of the cells caused by alpha-hemolysin and the inhibition of the antibody on alpha-hemolysin can be detected.

Hemolysis detection was performed on the culture supernatants of the 96-well plates after plate transferring, comprising following steps: the WT alpha-Toxin was diluted to obtain a stock solution with a concentration of 5 µg/mL; 25 µL of the stock solution was taken and mixed with an equal volume of cell culture supernatant, and the mixture was added into a 96-well plate containing 5% rabbit erythrocytes per well (diluted to 75 µL in PBS); and the 96-well plate was placed in an incubator at 37 °C for incubation for 1 h. Subsequently, the 96-well plate was centrifuged in a centrifuge at 3000 rpm for 3 min. 75 µL of the supernatant was added to a new 96-well plate and OD405 and OD450 were measured using a microplate reader. Part of the results is shown in panel 6B.

The screened hybridoma clones secreting anti-alpha-hemolysin antibodies were added into a 96-well plate which was plated with feeder cells by Limiting Dilution. Monoclonal cells were observed and marked under a microscope after 2-3 days, and monoclonal hybridoma cells capable of secreting anti-alpha-hemolysin monoclonal antibodies were screened by an ELISA assay after 7 days.

The monoclonal hybridoma cells secreting anti-alpha-hemolysin monoclonal antibodies were subjected to expanded culture, and total RNA of the cells was extracted using RNAfast200 Kit (Shanghai Flytech Biotechnology Co., Ltd.) according to the steps described in Kit instructions; the total RNA of the hybridoma cells obtained was reverse transcribed to cDNA using 5×PrimeScript RT Master Mix (Takara); and sequences of antibody light chain variable region, IgVL (κ) and heavy chain variable region V_{H} were amplified using degenerate primers (Anke Krebber., 1997) and Extaq PCR reagents (Takara). PCR amplification products were purified using PCR Clean-up Gel Extraction Kit (Macherey-Nagel GmbH & Co.); and linked to T-vector using pClone007 Simple Vector Kit (Tsingke Biotechnology Co., Ltd.) according to Kit instructions, and transformed into competent Escherichia coli cells. Variable region sequences of the monoclonal antibodies were obtained by DNA sequencing after strain amplification and plasmid extraction.

Variable region sequences of murine antibodies obtained were analyzed as follows:
> **murine antibody 98G9**
Heavy chain variable region: 
DVQLVESGGGLVQPGGSRKLSCAASGFTFSTFGMHWVRQAPEKGLEWVAYISGGSSTI
YYADTVKGRFTISRDNPKNTLFLQMTSLRSEDTAMYYCASGYPYGLDYWGQGTSVTV
SS (SEQ ID NO: 44)
Light chain variable region: 
DIDMTQSPSSMYASLGERVTITCKASQDINWYLSWFQQKPGKSPKTLIYRGNRLVDGV
PSRFSGSGSGQDYSLTISSLEYEDMGIYYCLQYDEFPFTFGSGTKLEIK (SEQ ID NO: 46)
Heavy and light chain CDRs were defined for murine antibody 98G9 using different definition tools, and are shown in Table 2.

**Table 2 CDR sequences of murine antibody 98G9**

| **Definition** | **Heavy chain CDR1** | **Heavy chain CDR2** | **Heavy chain CDR3** |
|---|---|---|---|
| Chothia | SEQ ID NO: 1 | SEQ ID NO: 4 | SEQ ID NO: 7 |
| AbM | SEQ ID NO: 2 | SEQ ID NO: 5 | SEQ ID NO: 7 |
| Kabat | SEQ ID NO: 3 | SEQ ID NO: 6 | SEQ ID NO: 7 |
| Contact | SEQ ID NO: 2 | SEQ ID NO: 6 | SEQ ID NO: 7 |

| **Definition** | **Light chain CDR1** | **Light chain CDR2** | **Light chain CDR3** |
|---|---|---|---|
| Chothia | SEQ ID NO: 8 | SEQ ID NO: 9 | SEQ ID NO: 10 |
| AbM | SEQ ID NO: 8 | SEQ ID NO: 9 | SEQ ID NO: 10 |
| Kabat | SEQ ID NO: 8 | SEQ ID NO: 9 | SEQ ID NO: 10 |
| Contact | SEQ ID NO: 8 | SEQ ID NO: 9 | SEQ ID NO: 10 |

> **murine antibody 78F4**
Heavy chain variable region: 
QVQLQQPGAELVRPGASVKLSCKASGYSFTSYWMNWVKQRPGQGLEWIGMIHPSDSE
TRLSQKFKDKATLTVDKSSSTAYMQLSSPTSEDSAVYYCTRFDWDRAMDYWGQGTSV
TVSS (SEQ ID NO: 52)
Light chain variable region: 
DIQMTQSPASLSASVGETVTITCRASENIFSYLAWYQQKQGKSPQLLVYNTRSLAEGVP
SRFSGSGSGTQFSLKINSLQPEDFGTYYCQHHYGTPWTFGGGTKLEIK (SEQ ID NO: 54)
Heavy and light chain CDRs were defined for murine antibody 78F4 using different definition tools, and are shown in Table 3.

**Table 3 CDR sequences of murine antibody 78F4**

| **Definition** | **Heavy chain CDR1** | **Heavy chain CDR2** | **Heavy chain CDR3** |
|---|---|---|---|
| Chothia | SEQ ID NO: 12 | SEQ ID NO: 15 | SEQ ID NO: 18 |
| AbM | SEQ ID NO: 13 | SEQ ID NO: 16 | SEQ ID NO: 18 |
| Kabat | SEQ ID NO: 14 | SEQ ID NO: 17 | SEQ ID NO: 18 |
| Contact | SEQ ID NO: 13 | SEQ ID NO: 17 | SEQ ID NO: 18 |

| **Definition** | **Light chain CDR1** | **Light chain CDR2** | **Light chain CDR3** |
|---|---|---|---|
| Chothia | SEQ ID NO: 19 | SEQ ID NO: 20 | SEQ ID NO: 21 |
| AbM | SEQ ID NO: 19 | SEQ ID NO: 20 | SEQ ID NO: 21 |
| Kabat | SEQ ID NO: 19 | SEQ ID NO: 20 | SEQ ID NO: 21 |
| Contact | SEQ ID NO: 19 | SEQ ID NO: 20 | SEQ ID NO: 21 |

> **murine antibody 78D4**
Heavy chain variable region: 
EVHLQQSGPELMKPGASVKISCKTSGYTFSEYTMHWVKQSHGKSLEWIGSINPNNGGT
TYNQKFKGKATLTVDKSSSTAYMELRSLTSEDSAVYNCARTRDYDNDGGLFAYWGQG
TLVTVSA (SEQ ID NO: 60)
Light chain variable region: 
DVQITQSPSYLAASPGETITINCRASKNISKYLAWYQEKPGKTNKLLIYSGSTLQSGIPSR
FSGNRSGTDFTLTISSLEPEDFAMYYCQQHYEYPFTFGGGTKLEIK (SEQ ID NO: 62)
Heavy and light chain CDRs were defined for murine antibody 78D4 using different definition tools, and are shown in Table 4.

**Table 4. CDR sequences of murine antibody 78D4**

| **Definition** | **Heavy chain CDR1** | **Heavy chain CDR2** | **Heavy chain CDR3** |
|---|---|---|---|
| Chothia | SEQ ID NO: 22 | SEQ ID NO: 25 | SEQ ID NO: 29 |
| AbM | SEQ ID NO: 23 | SEQ ID NO: 26 | SEQ ID NO: 29 |
| Kabat | SEQ ID NO: 24 | SEQ ID NO: 27 | SEQ ID NO: 29 |
| Contact | SEQ ID NO: 23 | SEQ ID NO: 27 | SEQ ID NO: 29 |

| **Definition** | **Light chain CDR1** | **Light chain CDR2** | **Light chain CDR3** |
|---|---|---|---|
| Chothia | SEQ ID NO: 30 | SEQ ID NO: 32 | SEQ ID NO: 33 |
| AbM | SEQ ID NO: 30 | SEQ ID NO: 32 | SEQ ID NO: 33 |
| Kabat | SEQ ID NO: 30 | SEQ ID NO: 32 | SEQ ID NO: 33 |
| Contact | SEQ ID NO: 30 | SEQ ID NO: 32 | SEQ ID NO: 33 |

> **murine antibody 16H4**
Heavy chain variable region: 
EVQLQQSGAELVKPGASVTLSCTVSGFNIKDTYMHWVKQRPEQGLEWIGKIDPASGNT
KYDPQFQGKATITADTSSNTAYLHLSSLTSEDSAVYFCASPYGNDYAMNYWGQGTSVT
VSS (SEQ ID NO: 68)
Light chain variable region: 
DIQMTQSPASLSASVGETVTITCRASEKIYSFLAWYQQKQEKSPQLLVYNAETLAEGVP
SRFSGTGSGIQFSLKIISLQPEDFGIYYCQHHYGTPYTFGGGTKLEIK (SEQ ID NO: 70)
Heavy and light chain CDRs were defined for murine antibody 16H4 using different definition tools, and are shown in Table 5.

**Table 5 CDR sequences of murine antibody 16H4**

| **Definition** | **Heavy chain CDR1** | **Heavy chain CDR2** | **Heavy chain CDR3** |
|---|---|---|---|
| Chothia | SEQ ID NO: 34 | SEQ ID NO: 37 | SEQ ID NO: 40 |
| AbM | SEQ ID NO: 35 | SEQ ID NO: 38 | SEQ ID NO: 40 |
| Kabat | SEQ ID NO: 36 | SEQ ID NO: 39 | SEQ ID NO: 40 |
| Contact | SEQ ID NO: 35 | SEQ ID NO: 39 | SEQ ID NO: 40 |

| **Definition** | **Light chain CDR1** | **Light chain CDR2** | **Light chain CDR3** |
|---|---|---|---|
| Chothia | SEQ ID NO: 41 | SEQ ID NO: 42 | SEQ ID NO: 43 |
| AbM | SEQ ID NO: 41 | SEQ ID NO: 42 | SEQ ID NO: 43 |
| Kabat | SEQ ID NO: 41 | SEQ ID NO: 42 | SEQ ID NO: 43 |
| Contact | SEQ ID NO: 41 | SEQ ID NO: 42 | SEQ ID NO: 43 |

### Example 8 Binding affinity of antibodies of the present invention to alpha-hemolysin (alpha-Toxin)

Alpha-hemolysin was diluted to 1 µg/mL with PBS, then 100 µL per well was added into a 96-well plate (Microwell 96F 167008, Thermo) and incubated at 4 °C overnight for coating. Next day, the 96-well plate was washed with PBST (containing 0.5% PBS), for which the wells were soaked with PBST for 1 min each time, and then residual water was thoroughly spun off. 200 µL of PBST containing 5% BSA was added into the wells of the plate respectively, which then was incubated at 37 °C for 1 h for blocking; subsequently, the plate was washed with PBST and then residual water in the wells was thoroughly spun off.

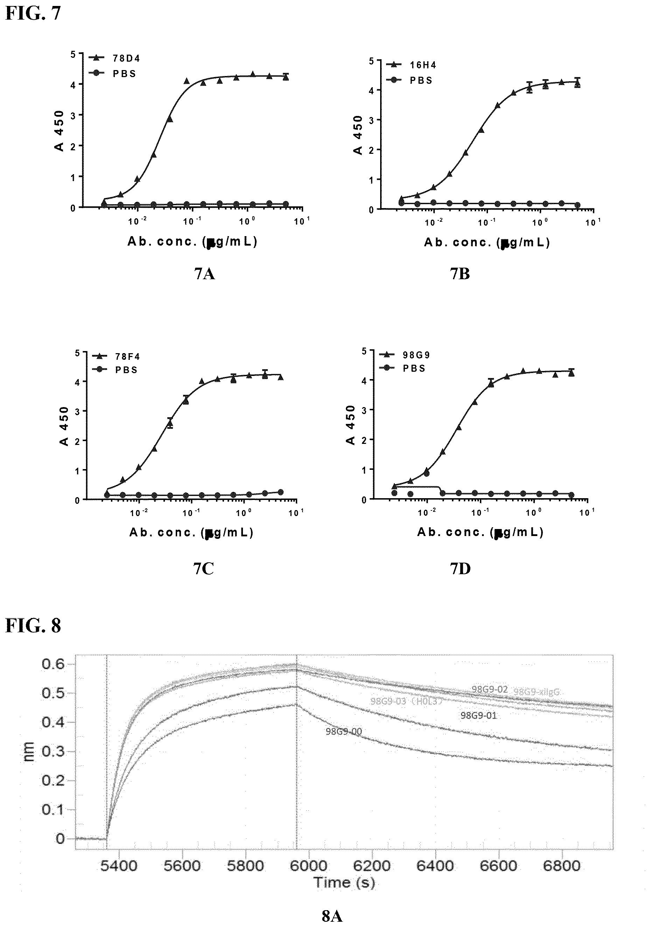

100 µL of each of recombinantly expressed anti-alpha-hemolysin monoclonal antibodies of different concentrations obtained through serial dilution (see the abscissae in FIG. 7 for antibody concentrations) was added to the 96-well plate per well, which then was incubated at 4 °C overnight. Then the 96-well plate was washed with PBST, before 100 µL of anti-mouse IgG secondary antibody (IH-0031, Beijing Dingguo Changsheng Biotechnology CO. LTD.) was added into each well, and the plate was incubated for 1 h at 37 °C. Washed with PBST for 5 times, the plate was added with 100 µL of Substrate Solution (Invitrogen) per well, and incubated at 37 °C for 10 min. The reaction was stopped by the addition of 50 µL of 2 N sulfuric acid per well, and absorbance at a wavelength of 450 nm was measured in a microplate reader (Multiskcin FC, Thermo).

The results are shown in FIG. 7.

### Example 9 Obtainment of chimeric antibodies and humanized antibodies of the present invention

First, complete light and heavy chain variable regions of the murine antibodies were combined with the human light and heavy chain constant regions to obtain chimeric antibodies as controls. Each obtained chimeric antibody was named following a format "murine antibody abbreviation-xi".

With a comprehensive analysis of the heavy chain sequence of each murine antibody, Complementarity Determining Regions (CDRs) of the antibody binding to the antigen as well as Framework Regions (FRs) supporting the conserved three-dimensional conformation of the antibody were determined. Sequences of known human antibodies were searched and analyzed, and a heavy chain sequence of human antibody which was most similar and closest to the murine antibody was selected, such as IGHV1-3^{∗}01, and the sequences of framework regions in it were selected as templates. The heavy chain CDRs of the murine antibody were grafted into the framework regions of the human antibody to generate a humanized antibody heavy chain sequence (heavy chain version 0). Subsequently, residues at individual amino acid positions corresponding to positions in the murine framework regions that may be involved in antigen-antibody binding were back mutated to original murine residues, to generate humanized antibody heavy chain sequences (versions 1, 2, 3, ......). Humanized antibody light chain sequences (version 0, 1, 2, ......) were generated by applying the same procedure. Humanized antibody light and heavy chain sequences were designed, synthesized, and co-transfected into HEK 293 cells and humanized antibodies were expressed recombinantly (versions were named as follows: e.g., H0L0 was obtained by co-expressing heavy chain version 0 + light chain version 0, and could be further abbreviated as version 00). Experiments proved that purified humanized antibodies showed activities of specifically binding to alpha-hemolysin protein consistent to those of murine parent antibodies respectively.

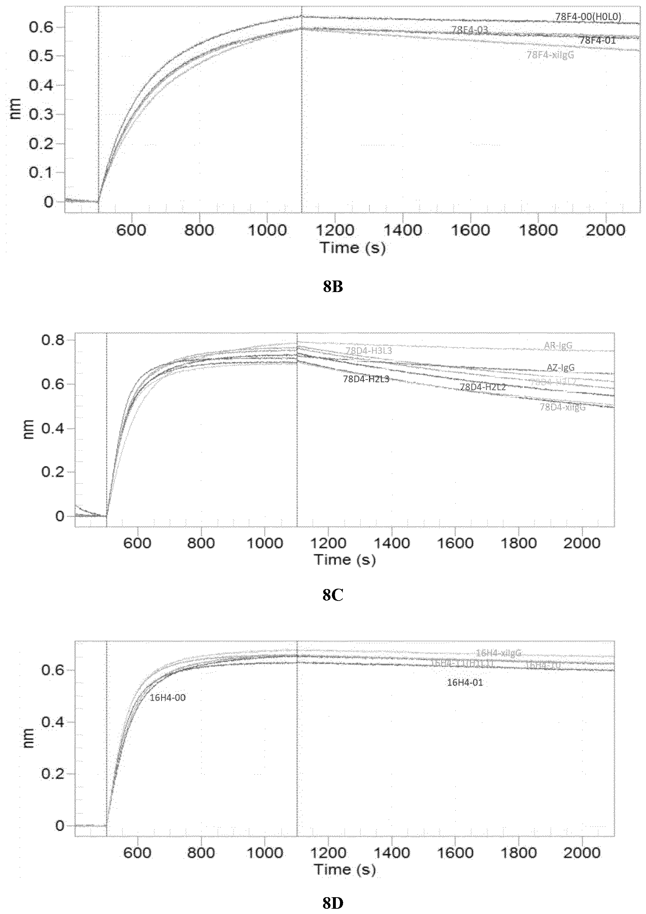

Those finally obtained humanized antibody versions and chimeric antibody xi versions were compared for antigen binding using an Octet instrument, and FIG. 8 shows measurement results of certain antibodies. From the curves of association and dissociation phases of binding of the antibodies to the antigen, certain humanized antibody versions shows similar or better properties in the antibody-antigen association and dissociation phases than the control antibodies (including chimeric antibodies of the present invention or antibodies AZ, and AR).

Humanized antibodies having following sequences were obtained by screening.
> **humanized antibody 98G9-02**(98G9-H0L2)
   Heavy chain variable region (H0): 
EVQLVESGGGLVQPGGSLRLSCAASGFTFSTFGMHWVRQAPGKGLEWVSYISGGSSTI
YYADTVKGRFTISRDNAKNSLYLQMNSLRAEDTAVYYCASGYPYGLDYWGQGTLVTV
SS (SEQ ID NO: 48)
   Light chain variable region (L2): 
DIQMTQSPSSLSASVGDRVTITCKASQDINWYLSWFQQKPGKAPKTLIYRGNRLVDGV
PSRFSGSGSGQDYTFTISSLQPEDMATYYCLQYDEFPFTFGQGTKVEIK (SEQ ID NO:
76)
> **humanized antibody98G9-03** (98G9-H0L3)
   Heavy chain variable region (H0): 
EVQLVESGGGLVQPGGSLRLSCAASGFTFSTFGMHWVRQAPGKGLEWVSYISGGSSTI
YYADTVKGRFTISRDNAKNSLYLQMNSLRAEDTAVYYCASGYPYGLDYWGQGTLVTV
SS (SEQ ID NO: 48)
   Light chain variable region (L3): 
DIQMTQSPSSLSASVGDRVTITCKASQDINWYLSWFQQKPGKAPKTLIYRGNRLVEGV
PSRFSGSGSGQDYTFTISSLQPEDMATYYCLQYDEFPFTFGQGTKVEIK (SEQ ID NO:
50)
> **humanized antibody78F4-00** (78F4-H0L0)
   Heavy chain variable region (H0): QVQLVQSGAEVKKPGASVKVSCKASGYSFTSYWMNWVRQAPGQGLEWMGMIHPSD 
SETRLSQKFKDRVTMTRDTSTSTVYMELSSLRSEDTAVYYCARFDWDRAMDYWGQG
TLVTVSS (SEQ ID NO: 56)
   Light chain variable region (L0): 
DIQMTQSPSSLSASVGDRVTITCRASENIFSYLAWYQQKPGKAPKLLIYNTRSLAEGVPS
RFSGSGSGTDFTLTISSLQPEDFATYYCQHHYGTPWTFGQGTKLEIK (SEQ ID NO: 58)
> **humanized** antibody78D4-33 (78D4-H3L3)
   Heavy chain variable region (H3): 
QVQLVQSGAEVKKPGASVKVSCKTSGYTFSEYTMHWVRQAPGQRLEWMGSINPNQG
GTTYNQKFKGRVTITVDKSASTAYMELSSLRSEDTAVYYCARTRDYDNDGGLFAYWG
QGTLVTVSS (SEQ ID NO: 64)
   Light chain variable region (L3): 
DVQITQSPSFLSASVGDRVTITCRASKNILKYLAWYQQKPGKAPKLLIYSGSTLQSGVPS
RFSGSRSGTEFTLTISSLQPEDFATYYCQQHYEYPFTFGQGTKLEIK (SEQ ID NO: 66)
> **humanized antibody16H4-11** (16H4-H1L1)
   Heavy chain variable region (H1): 
EVQLVQSGAEVKKPGATVKISCKVSGFNIKDTYMHWVQQAPGKGLEWMGKIDPASG
NTKYDPQFQGRVTITADTSTNTAYMELSSLRSEDTAVYYCATPYGNDYAMNYWGQGT
LVTVSS (SEQ ID NO: 72)
   Light chain variable region (L1):

DIQMTQSPSSLSASVGDRVTITCRASEKIYSFLAWYQQKPGKAPKLLLYNAETLAEGVP
SRFSGSGSGIDFTLTISSLQPEDFATYYCQHHYGTPYTFGGGTKVEIK (SEQ ID NO: 74)

Heavy and light chain CDRs of the humanized antibodies are shown in Table 6.

**Table 6 CDR sequences of humanized antibodies**

| | | | |
|---|---|---|---|
| 98G9 H0L2 | heavy chain CDR1 | heavy chain CDR2 | heavy chain CDR3 |
| | SEQ ID NO: 2 | SEQ ID NO: 6 | SEQ ID NO: 7 |
| | light chain CDR1 | light chain CDR2 | light chain CDR3 |
| | SEQ ID NO: 8 | SEQ ID NO: 9 | SEQ ID NO: 10 |
| 98G9 H0L3 | heavy chain CDR1 | heavy chain CDR2 | heavy chain CDR3 |
| | SEQ ID NO: 2 | SEQ ID NO: 6 | SEQ ID NO: 7 |
| | light chain CDR1 | light chain CDR2 | light chain CDR3 |
| | SEQ ID NO: 8 | SEQ ID NO: 11 | SEQ ID NO: 10 |
| 78F4 H0L0 | heavy chain CDR1 | heavy chain CDR2 | heavy chain CDR3 |
| | SEQ ID NO: 13 | SEQ ID NO: 17 | SEQ ID NO: 18 |
| | light chain CDR1 | light chain CDR2 | light chain CDR3 |
| | SEQ ID NO: 19 | SEQ ID NO: 20 | SEQ ID NO: 21 |
| 78D4 H3L3 | heavy chain CDR1 | heavy chain CDR2 | heavy chain CDR3 |
| | SEQ ID NO: 23 | SEQ ID NO: 28 | SEQ ID NO: 29 |
| | light chain CDR1 | light chain CDR2 | light chain CDR3 |
| | SEQ ID NO: 31 | SEQ ID NO: 32 | SEQ ID NO: 33 |
| 16H4 H1L1 | heavy chain CDR1 | heavy chain CDR2 | heavy chain CDR3 |
| | SEQ ID NO: 35 | SEQ ID NO: 39 | SEQ ID NO: 40 |
| | light chain CDR1 | light chain CDR2 | light chain CDR3 |
| | SEQ ID NO: 41 | SEQ ID NO: 42 | SEQ ID NO: 43 |

### Example 10 Detection of antihemolytic activity of antibodies of the present invention against alpha-hemolysin (alpha-Toxin)

75 µL of 5% rabbit erythrocytes (Bio-channel Biotechnology) and 12.5 ng Staphylococcus aureus alpha-hemolysin and different amounts of anti-alpha-hemolysin antibodies (12.5 ng, 25ng, and 50 ng) were added to the wells of a 96-well plate, and PBS was added to obtain a final volume of 150 µL. The 96-well plate was incubated in an incubator at 37 °C for 1 h, and then was centrifuged at 3000 rpm for 3 min. 100 µL of the supernatant from each well was evaluated for hemolytic activity by absorbance at 405 nm (OD405) measured using a microplate reader.

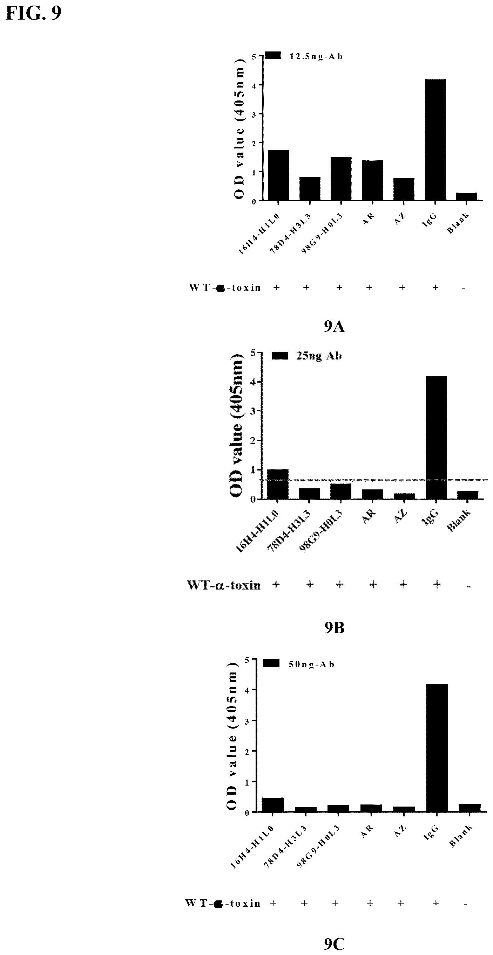

The experimental results showed that the chimeric antibodies and humanized antibodies of the present invention had obvious antihemolytic activities against alpha-hemolysin, which were dose dependent, and certain antibodies had comparable activities to control antibodies AR and AZ. The results are shown in FIG. 9.

### Example 11 Detection of binding kinetics (Kₒₙ, K_{off}) and affinity constant KD of antibodies of the present invention to alpha-hemolysin (alpha-Toxin)

Antibody-antigen interactions were measured using BIAcore S200 from GE Healthcare.

Referring to the instructions provided in Human Antibody Capture Kit (cat No. BR-1008-39, Lot 10261753) from GE Healthcare, the analytical channel and the control sample channel on CM5 sensor chip were first saturated and coupled with maximum amount of anti-human Fc antibody, then the antibody to be detected at 7.5 ug/ml was allowed to flow through the analytical channel and uniformly distribute; and finally antigen samples gradiently diluted (the initial concentration was 20 nM and diluted by 1:3 to get 8 concentrations, and the concentration 0.741 nM was set to be repeated) were allowed to flow through both the analytical channel and the sample channel, and the photoreactions upon the binding of antibody to antigen were measured. Subsequently, the association constant Kon and dissociation constant Koff and affinity constant KD of the antibody were finally obtained by instrument software fitting (1:1) analysis.

The results are shown in Table 7.

**Table 7 Binding kinetics and affinity constants of antibodies of the present invention to antigen**

| Antibody | ka (1/Ms) | kd (1/s) | KD (M) | Rmax (RU) | Chi² (RU²) |
|---|---|---|---|---|---|
| 78D4 xiIgG | 1.40E+06 | 3.36E-04 | 2.39E-10 | 67.1 | 0.198 |
| 78D4-H3L3 | 1.25E+06 | 3.02E-04 | 2.40E-10 | 37.2 | 0.0303 |
| 98G9 xiIgG | 8.84E+05 | 3.77E-04 | 4.26E-10 | 47.3 | 0.46 |
| 98G9-H0L2 | 7.76E+05 | 5.24E-04 | 6.74E-10 | 18.9 | 0.0677 |
| 98G9-H0L3 | 7.19E+05 | 5.12E-04 | 7.13E-10 | 22.5 | 0.0481 |
| AZ IgG | 1.60E+06 | 2.11E-04 | 1.32E-10 | 48.8 | 0.0552 |
| AR IgG | 2.76E+05 | 6.77E-05 | 2.45E-10 | 48.7 | 0.48 |

### Example 12 Establishment of animal model of alpha-hemolysin-induced sepsis and detection of therapeutic effects of antibodies of the present invention

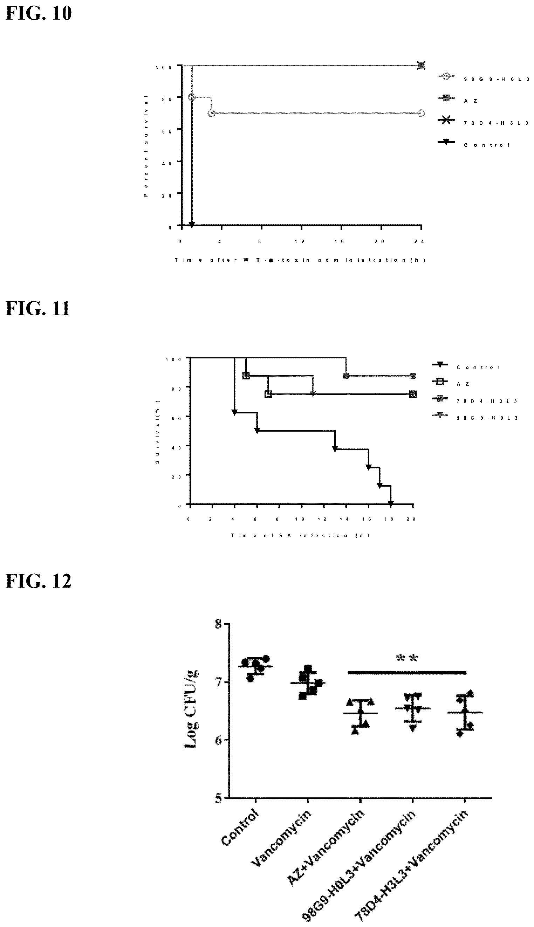

C57BL/6J mice were randomly divided into a model control group and monoclonal antibody drug treatment groups according to body weight. 30 min before experiment, mice in the treatment groups were injected with a corresponding anti-alpha-hemolysin monoclonal antibody (6 µg/mouse) via tail vein respectively, and mice in the control group were injected with the same dose of PBS; and then all the mice were injected with alpha-hemolysin (3 µg/mouse) via tail vein to establish a sepsis infected mouse model. The survival time of the experimental animals was observed and recorded, and the results are shown in FIG. 10.

### Example 13 Establishment of animal model of methicillin-resistant Staphylococcus aureus-induced bacteremia and detection of therapeutic effects of antibodies of the present invention

A methicillin-resistant Staphylococcus aureus USA300 strain was activated for 2 generations on a solid TSB plate, and then inoculated into a liquid TSB medium for overnight culture. The bacterial cells were collected by being centrifuged at 12,000 rpm, and resuspended in physiological saline for later use.

C57BL/6J mice were infected with 6×10⁷ CFU/mouse USA300 via tail vein and were randomly divided into a model control group (Control) and anti-alpha-hemolysin monoclonal antibody drug treatment groups according to body weight. 2 h after infection, the mice in the monoclonal antibody drug treatment groups were administered by tail vein injection a dosage of 5 mg/kg of a corresponding antibody respectively, and the mice in the control group were injected the same dose of PBS. The survival time of the mice in the groups was observed and recorded, and the results are shown in FIG. 11.

### Example 14 Establishment of animal model of methicillin-resistant Staphylococcus aureus pneumonia and detection of therapeutic effects of antibodies of the present invention

A methicillin-resistant Staphylococcus aureus USA300 strain was activated for 2 generations on a solid TSB plate, and then inoculated into a liquid TSB medium for overnight culture. The bacterial cells were collected by being centrifuged at 12,000 rpm, and resuspended in physiological saline for later use.

C57BL/6J mice were infected with 1.8×10⁸ CFU/mouse USA300 via trachea and were randomly divided into a model control group, a monoclonal antibody drug treatment group, a vancomycin treatment group, and vancomycin plus monoclonal antibody treatment groups according to body weight. 2 h after infection, the mice in the groups were administered by tail vein injection corresponding drug for treatment (the dosage of the corresponding monoclonal antibody was 15 mg/kg, and the dosage of vancomycin was 1.25 mg/kg), or the same volume of PBS. The animals were killed 24 h after infection, and lung tissues were taken, weighted and homogenized, and coated on a solid TSB medium to detect bacterial load in the tissues. The experimental results showed that the anti-alpha-hemolysin antibodies of the present invention were capable of enhancing the pharmacodynamic effect of vancomycin in the treatment of methicillin-resistant Staphylococcus aureus pneumonia.

The results are shown in FIG. 12.

### Example 15 Acute toxicity study of antibodies of the present invention

Acute toxicity study on antibody molecule 78D4 H3L3 of the present invention was performed in mice and cynomolgus monkeys (N = 10 for the experiment in mice, and N = 3 for the experiment in cynomolgus monkeys).

C57BL/6 mice (18-20 g), half male and half female, were injected with 125 mg/kg of antibody molecule 78D4 H3L3 per mouse via tail vein within 24 hours. The results showed that when a dosage of 125 mg/kg at the maximum was administered in 24 hours, no mice died, and no discomfort was shown by the animals during continuous observation for 14 days, and no abnormality was seen in main organs (heart, liver, spleen, lung, kidney and brain) taken for histology observation from sacrificed animals.

Male cynomolgus monkeys were administered 10 mg/kg of antibody molecule 78D4 H3L3 per monkey via intravenous drip in limbs, in a single dose. The results showed that when a dosage of 10 mg/kg of antibody molecule 78D4 H3L3 was administrated, no death of the animals occurred, and no discomfort was shown by the animals during continuous observation for 28 days.

Acute toxicity test research showed that antibody molecule 78D4 H3L3 had good safety.

### Example 16 Pharmacokinetic study of antibodies of the present invention

Antibody molecule 78D4 H3L3, at a dose of 10 mg/kg, was subjected to a single dose pharmacokinetic study in cynomolgus monkeys (N = 3).

Male cynomolgus monkeys were administered 10 mg/kg of antibody molecule 78D4 H3L3 per monkey via intravenous drip in limbs, in a single dose. Blood was sampled at 0 h before administration (pre-dose, 0 h), and at 0.25 h (15 min), 0.5 h (time point when needle was withdrawn), 4 h, 24 h (D2), 48 h (D3), 96 h (D5), 168 h (D8), 336 h (D15), 504 h (D22), and 672 h (D29) after administration started. Blood sampling sites were in peripheral veins in limbs of the animals (not the administered limb), or inguinal veins of the animals. The blood samples were about 1 mL whole blood/animal/time point. Antibody concentrations in the sera from the cynomolgus monkeys were determined using ELISA, and pharmacokinetic parameters such as AUClast, CL, T1/2 and the like were calculated by Phoenix WinNonlin (v6.4, Pharsight, Inc.) software using non-compartmental analysis methods.

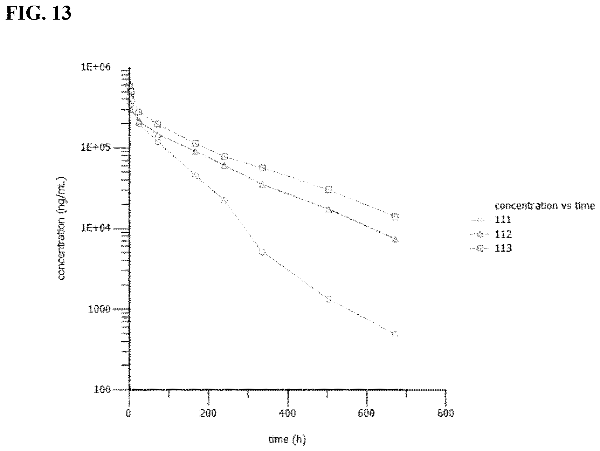

The concentrations of antibody 78D4 H3L3 in sera from the cynomolgus monkeys were determined using ELISA. Serum drug levels of individual animals are shown in Fig. 13, and pharmacokinetic parameters are summarized in Table 8. Drug exposure can be seen with each animal after administration.

**Table 8 Pharmacokinetic results of antibody 78D4 H3L3**

| | | **Animal ID** | | | | | | |
|---|---|---|---|---|---|---|---|---|
| | | **111** | **112** | **113** | **N** | **Mean** | **SD** | **CV%** |
| **Parameter** | **Unit** | **Estimate** | | | | | | |
| AUC_{INF} obs | h^{∗}ng/mL | 26900000 | 44700000 | 64300000 | 3 | 45300000 | 18700000 | 41.3 |
| AUCₗₐₛₜ | h^{∗}ng/mL | 26800000 | 43200000 | 60700000 | 3 | 43600000 | 17000000 | 38.9 |
| C₀ | ng/mL | 431000 | 387000 | 592000 | 3 | 470000 | 108000 | 22.9 |
| Cl _{obs} | mL/h/kg | 0.744 | 0.447 | 0.311 | 3 | 0.501 | 0.222 | 44.2 |
| MRTₗₐₛₜ | h | 83.8 | 160 | 170 | 3 | 138 | 47.1 | 34.2 |
| T_{1/2z} | h | 99.2 | 140 | 173 | 3 | 137 | 36.9 | 26.9 |
| V_{z_obs} | mL/kg | 107 | 90.4 | 77.6 | 3 | 91.5 | 14.5 | 15.8 |

The above description of the embodiments of the present invention is not intended to limit the present invention, and those skilled in the art may make various changes and modifications to the present invention without departing from the spirit of the present invention, which should fall within the scope of the appended claims.

## Claims

1. A method for preparing an anti-alpha-hemolysin monoclonal antibody, including steps as follows:
(1) immunizing animals with an attenuated alpha-hemolysin or an alpha-hemolysin without virulence;
(2) taking splenocytes and preparing hybridomas;
(3) screening an antibody having high affinity to virulent alpha-hemolysin or wild-type alpha-hemolysin using the same;
(4) selecting an antibody which has an activity of inhibiting erythrocyte lysis caused by virulent alpha-hemolysin or wild-type alpha-hemolysin through an assay of inhibition of erythrocyte lysis.

2. The method for preparing an anti-alpha-hemolysin monoclonal antibody according to claim 1, wherein the virulent alpha-hemolysin or wild-type alpha-hemolysin is Staphylococcus aureus alpha-hemolysin, and preferably has an amino acid sequence as shown in SEQ ID NO: 78; and the attenuated alpha-hemolysin or alpha-hemolysin without virulence is alpha-hemolysin with H35L, and preferably has an amino acid sequence as shown in SEQ ID NO: 80.

3. The method for preparing an anti-alpha-hemolysin monoclonal antibody according to claim 1, wherein the virulent alpha-hemolysin or wild-type alpha-hemolysin, the attenuated alpha-hemolysin, or the alpha-hemolysin without virulence is independently linked to a purification tag, a carrier protein, and/or an adjuvant molecule; and the linkage includes coupling, cross-linking, conjugation, and/or fusion.

4. An anti-alpha-hemolysin monoclonal antibody prepared by the method according to any one of claims 1 to 3, wherein the antibody has at least one activity selected from the group consisting of blocking hemolytic effect of alpha-hemolysin, blocking damage of alpha-hemolysin to lung epithelial cells, reducing bacterial load in MRSA infected lung tissue, prolonging survival time of a patient suffering from MRSA bacteremia, and preventing or alleviating sepsis caused by alpha-hemolysin.

5. An anti-alpha-hemolysin monoclonal antibody prepared by the method according to any one of claims 1 to 3, wherein the antibody has
VH-CDR1: selected from the group consisting of SEQ ID NOs: 1, 2, 3, 12, 13, 14, 22, 23, 24, 34, 35, and 36;
VH-CDR2: selected from the group consisting of SEQ ID NOs: 4, 5, 6, 15, 16, 17, 25, 26, 27, 28, 37, 38, and 39;
VH-CDR3: selected from the group consisting of SEQ ID NOs: 7, 18, 29, and 40;
VL-CDR1: selected from the group consisting of SEQ ID NOs: 8, 19, 30, 31, and 41;
VL-CDR2: selected from the group consisting of SEQ ID NOs: 9, 11, 20, 32, and 42; and
VL-CDR3: selected from the group consisting of SEQ ID NOs: 10, 21, 33, and 43.

6. An antibody or fragment thereof comprising a heavy chain variable region (VH) and a light chain variable region (VL), wherein the heavy chain variable region (VH) and the light chain variable region (VL) comprise a combination of CDRs (VH-CDR1, VH-CDR2, VH-CDR3; and VL-CDR1, VL-CDR2, VL-CDR3) selected from the group consisting of:
(1) VH-CDR1 as shown in SEQ ID NO: 1, VH-CDR2 as shown in SEQ ID NO: 4, and VH-CDR3 as shown in SEQ ID NO: 7; and, VL-CDR1 as shown in SEQ ID NO: 8, VL-CDR2 as shown in SEQ ID NO: 9, and VL-CDR3 as shown in SEQ ID NO: 10;
(2) VH-CDR1 as shown in SEQ ID NO: 2, VH-CDR2 as shown in SEQ ID NO: 5, and VH-CDR3 as shown in SEQ ID NO: 7; and, VL-CDR1 as shown in SEQ ID NO: 8, VL-CDR2 as shown in SEQ ID NO: 9, and VL-CDR3 as shown in SEQ ID NO: 10;
(3) VH-CDR1 as shown in SEQ ID NO: 3, VH-CDR2 as shown in SEQ ID NO: 6, and VH-CDR3 as shown in SEQ ID NO: 7; and, VL-CDR1 as shown in SEQ ID NO: 8, VL-CDR2 as shown in SEQ ID NO: 9, and VL-CDR3 as shown in SEQ ID NO: 10;
(4) VH-CDR1 as shown in SEQ ID NO: 2, VH-CDR2 as shown in SEQ ID NO: 6, and VH-CDR3 as shown in SEQ ID NO: 7; and, VL-CDR1 as shown in SEQ ID NO: 8, VL-CDR2 as shown in SEQ ID NO: 9, and VL-CDR3 as shown in SEQ ID NO: 10;
(5) VH-CDR1 as shown in SEQ ID NO: 2, VH-CDR2 as shown in SEQ ID NO: 6, and VH-CDR3 as shown in SEQ ID NO: 7; and, VL-CDR1 as shown in SEQ ID NO: 8, VL-CDR2 as shown in SEQ ID NO: 11, and VL-CDR3 as shown in SEQ ID NO: 10;
(6) VH-CDR1 as shown in SEQ ID NO: 12, VH-CDR2 as shown in SEQ ID NO: 15, and VH-CDR3 as shown in SEQ ID NO: 18; and, VL-CDR1 as shown in SEQ ID NO: 19, VL-CDR2 as shown in SEQ ID NO: 20, and VL-CDR3 as shown in SEQ ID NO: 21;
(7) VH-CDR1 as shown in SEQ ID NO: 13, VH-CDR2 as shown in SEQ ID NO: 16, and VH-CDR3 as shown in SEQ ID NO: 18; and, VL-CDR1 as shown in SEQ ID NO: 19, VL-CDR2 as shown in SEQ ID NO: 20, and VL-CDR3 as shown in SEQ ID NO: 21;
(8) VH-CDR1 as shown in SEQ ID NO: 14, VH-CDR2 as shown in SEQ ID NO: 17, and VH-CDR3 as shown in SEQ ID NO: 18; and, VL-CDR1 as shown in SEQ ID NO: 19, VL-CDR2 as shown in SEQ ID NO: 20, and VL-CDR3 as shown in SEQ ID NO: 21;
(9) VH-CDR1 as shown in SEQ ID NO: 13, VH-CDR2 as shown in SEQ ID NO: 17, and VH-CDR3 as shown in SEQ ID NO: 18; and, VL-CDR1 as shown in SEQ ID NO: 19, VL-CDR2 as shown in SEQ ID NO: 20, and VL-CDR3 as shown in SEQ ID NO: 21;
(10) VH-CDR1 as shown in SEQ ID NO: 22, VH-CDR2 as shown in SEQ ID NO: 25, and VH-CDR3 as shown in SEQ ID NO: 29; and, VL-CDR1 as shown in SEQ ID NO: 30, VL-CDR2 as shown in SEQ ID NO: 32, and VL-CDR3 as shown in SEQ ID NO: 33;
(11) VH-CDR1 as shown in SEQ ID NO: 23, VH-CDR2 as shown in SEQ ID NO: 26, and VH-CDR3 as shown in SEQ ID NO: 29; and, VL-CDR1 as shown in SEQ ID NO: 30, VL-CDR2 as shown in SEQ ID NO: 32, and VL-CDR3 as shown in SEQ ID NO: 33;
(12) VH-CDR1 as shown in SEQ ID NO: 24, VH-CDR2 as shown in SEQ ID NO: 27, and VH-CDR3 as shown in SEQ ID NO: 29; and, VL-CDR1 as shown in SEQ ID NO: 30, VL-CDR2 as shown in SEQ ID NO: 32, and VL-CDR3 as shown in SEQ ID NO: 33;
(13) VH-CDR1 as shown in SEQ ID NO: 23, VH-CDR2 as shown in SEQ ID NO: 27, and VH-CDR3 as shown in SEQ ID NO: 29; and, VL-CDR1 as shown in SEQ ID NO: 30, VL-CDR2 as shown in SEQ ID NO: 32, and VL-CDR3 as shown in SEQ ID NO: 33;
(14) VH-CDR1 as shown in SEQ ID NO: 23, VH-CDR2 as shown in SEQ ID NO: 28, and VH-CDR3 as shown in SEQ ID NO: 29; and, VL-CDR1 as shown in SEQ ID NO: 31, VL-CDR2 as shown in SEQ ID NO: 32, and VL-CDR3 as shown in SEQ ID NO: 33;
(15) VH-CDR1 as shown in SEQ ID NO: 34, VH-CDR2 as shown in SEQ ID NO: 37, and VH-CDR3 as shown in SEQ ID NO: 40; and, VL-CDR1 as shown in SEQ ID NO: 41, VL-CDR2 as shown in SEQ ID NO: 42, and VL-CDR3 as shown in SEQ ID NO: 43;
(16) VH-CDR1 as shown in SEQ ID NO: 35, VH-CDR2 as shown in SEQ ID NO: 38, and VH-CDR3 as shown in SEQ ID NO: 40; and, VL-CDR1 as shown in SEQ ID NO: 41, VL-CDR2 as shown in SEQ ID NO: 42, and VL-CDR3 as shown in SEQ ID NO: 43;
(17) VH-CDR1 as shown in SEQ ID NO: 36, VH-CDR2 as shown in SEQ ID NO: 39, and VH-CDR3 as shown in SEQ ID NO: 40; and, VL-CDR1 as shown in SEQ ID NO: 41, VL-CDR2 as shown in SEQ ID NO: 42, and VL-CDR3 as shown in SEQ ID NO: 43; and
(18) VH-CDR1 as shown in SEQ ID NO: 35, VH-CDR2 as shown in SEQ ID NO: 39, and VH-CDR3 as shown in SEQ ID NO: 40; and, VL-CDR1 as shown in SEQ ID NO: 41, VL-CDR2 as shown in SEQ ID NO: 42, and VL-CDR3 as shown in SEQ ID NO: 43.

7. The antibody or fragment thereof according to any one of claims 4 to 6, wherein the heavy chain variable region comprises:
an amino acid sequence as shown in any one of SEQ ID NO: 44, SEQ ID NO: 48, SEQ ID NO: 52, SEQ ID NO: 56, SEQ ID NO: 60, SEQ ID NO: 64, SEQ ID NO: 68 and SEQ ID NO: 72, or an amino acid sequence having at least 75% identity to the amino acid sequence as shown; and/or
the light chain variable region comprises:
an amino acid sequence as shown in any one of SEQ ID NO: 46, SEQ ID NO: 50, SEQ ID NO: 54, SEQ ID NO: 58, SEQ ID NO: 62, SEQ ID NO: 66, SEQ ID NO: 70, SEQ ID NO: 74 and SEQ ID NO: 76 or an amino acid sequence having at least 75% identity to the amino acid sequence as shown.

8. The antibody or fragment thereof according to any one of claims 4 to 7, wherein the antibody or fragment thereof comprises a heavy chain variable region and a light chain variable region selected from the group consisting of following combinations:
(1) an amino acid sequence as shown in SEQ ID NO: 44 or an amino acid sequence having at least 75% identity to the amino acid sequence as shown in SEQ ID NO: 44; and, an amino acid sequence as shown in SEQ ID NO: 46 or an amino acid sequence having at least 75% identity to the amino acid sequence as shown in SEQ ID NO: 46;
(2) an amino acid sequence as shown in SEQ ID NO: 48 or an amino acid sequence having at least 75% identity to the amino acid sequence as shown in SEQ ID NO: 48; and, an amino acid sequence as shown in SEQ ID NO: 50 or an amino acid sequence having at least 75% identity to the amino acid sequence as shown in SEQ ID NO: 50;
(3) an amino acid sequence as shown in SEQ ID NO: 52 or an amino acid sequence having at least 75% identity to the amino acid sequence as shown in SEQ ID NO: 52; and, an amino acid sequence as shown in SEQ ID NO: 54 or an amino acid sequence having at least 75% identity to the amino acid sequence as shown in SEQ ID NO: 54;
(4) an amino acid sequence as shown in SEQ ID NO: 56 or an amino acid sequence having at least 75% identity to the amino acid sequence as shown in SEQ ID NO: 56; and, an amino acid sequence as shown in SEQ ID NO: 58 or an amino acid sequence having at least 75% identity to the amino acid sequence as shown in SEQ ID NO: 58;
(5) an amino acid sequence as shown in SEQ ID NO: 60 or an amino acid sequence having at least 75% identity to the amino acid sequence as shown in SEQ ID NO: 60; and, an amino acid sequence as shown in SEQ ID NO: 62 or an amino acid sequence having at least 75% identity to the amino acid sequence as shown in SEQ ID NO: 62;
(6) an amino acid sequence as shown in SEQ ID NO: 64 or an amino acid sequence having at least 75% identity to the amino acid sequence as shown in SEQ ID NO: 64; and, an amino acid sequence as shown in SEQ ID NO: 66 or an amino acid sequence having at least 75% identity to the amino acid sequence as shown in SEQ ID NO: 66;
(7) an amino acid sequence as shown in SEQ ID NO: 68 or an amino acid sequence having at least 75% identity to the amino acid sequence as shown in SEQ ID NO: 68; and, an amino acid sequence as shown in SEQ ID NO: 70 or an amino acid sequence having at least 75% identity to the amino acid sequence as shown in SEQ ID NO: 70;
(8) an amino acid sequence as shown in SEQ ID NO: 72 or an amino acid sequence having at least 75% identity to the amino acid sequence as shown in SEQ ID NO: 72; and, an amino acid sequence as shown in SEQ ID NO: 74 or an amino acid sequence having at least 75% identity to the amino acid sequence as shown in SEQ ID NO: 74; and
(9) an amino acid sequence as shown in SEQ ID NO: 48 or an amino acid sequence having at least 75% identity to the amino acid sequence as shown in SEQ ID NO: 48; and, an amino acid sequence as shown in SEQ ID NO: 76 or an amino acid sequence having at least 75% identity to the amino acid sequence as shown in SEQ ID NO: 76.

9. The antibody or fragment thereof according to any one of claims 4 to 8, wherein the at least 75% identity is any percent identity greater than or equal to 75%, such as at least 80%, preferably at least 85%, more preferably at least 90%, even more preferably at least 91%, 92%, 93%, 94%, 95%, 96%, 97%, 98%, or even 99% identity;
preferably, the antibody or fragment thereof is in any form, for example, a monoclonal antibody, a single-chain antibody, a single-domain antibody, a bifunctional antibody, a nanobody, a fully or partially humanized antibody or a chimeric antibody and the like; alternatively, the antibody or fragment thereof is a half-antibody or an antigen-binding fragment of a half-antibody, for example, scFv, BsFv, dsFv, (dsFv)₂, Fab, Fab', F(ab')₂, or Fv; alternatively, the antibody is IgA, IgD, IgE, IgG or IgM, more preferably IgG1; or, the fragment of the antibody is selected from the group consisting of scFv, Fab, F(ab')₂ and Fv fragments of the antibody.

10. The antibody or fragment thereof according to any one of claims 4 to 9, wherein the antibody or fragment thereof further comprises a human or murine light chain constant region (CL) and/or heavy chain constant region (CH); preferably, the antibody or fragment thereof comprises a heavy chain constant region selected from the group consisting of IgG, IgA, IgM, IgD and IgE and/or a kappa or lambda type light chain constant region; more preferably, the heavy chain constant region is of IgG1 or IgG4 subtype and the light chain constant region is of kappa type.

11. A nucleic acid molecule comprising a nucleotide sequence encoding the antibody or fragment thereof according to any one of claims 4 to 10, or encoding a heavy chain CDR, a light chain CDR, a heavy chain variable region, a light chain variable region, a heavy chain or a light chain comprised in the antibody or fragment thereof.

12. A vector comprising the nucleic acid molecule according to claim 11.

13. A host cell comprising the nucleic acid molecule according to claim 11 and/or the vector according to claim 12, or transformed or transfected with the nucleic acid molecule according to claim 11 and/or the vector according to claim 12.

14. A conjugate or fusion protein comprising the antibody or fragment thereof according to any one of claims 4 to 10.

15. The conjugate or fusion protein according to claim 14, wherein the conjugate or fusion protein comprises an additional moiety, for example, a cell surface receptor, a small molecule compound, a small molecule polymer, an active protein or a polypeptide, directly or indirectly linked to the antibody or fragment thereof.

16. A pharmaceutical composition comprising the antibody or fragment thereof according to any one of claims 4 to 10, the nucleic acid molecule according to claim 11, the vector according to claim 12, the host cell according to claim 13, and/or the conjugate or fusion protein according to claim 14 or 15, and optionally a pharmaceutically acceptable excipient.

17. A kit comprising the antibody or fragment thereof according to any one of claims 4 to 10, the nucleic acid molecule according to claim 11, the vector according to claim 12, the host cell according to claim 13, the conjugate or fusion protein according to claim 14 or 15, and/or the pharmaceutical composition according to claim 16.

18. Use of the antibody or fragment thereof according to any one of claims 4 to 10, the nucleic acid molecule according to claim 11, the vector according to claim 12, the host cell according to claim 13, the conjugate or fusion protein according to claim 14 or 15, and/or the pharmaceutical composition according to claim 16 in the manufacture of a medicament for preventing or treating infection and complication caused by alpha-hemolysin or an alpha-hemolysin producing microorganism.

19. Use of the antibody or fragment thereof according to any one of claims 4 to 10, the nucleic acid molecule according to claim 11, the vector according to claim 12, the host cell according to claim 13, the conjugate or fusion protein according to claim 14 or 15, and/or the pharmaceutical composition according to claim 16 in combination with another antibacterial agent or anti-alpha-hemolysin antibody in the manufacture of a medicament for preventing or treating infection and complication caused by alpha-hemolysin or an alpha-hemolysin producing microorganism.

20. Use according to claim 18 or 19, wherein the microorganism is Staphylococcus aureus;
preferably, the Staphylococcus aureus includes methicillin-resistant Staphylococcus aureus;
preferably, the infection includes upper respiratory tract infection, pneumonia, severe pneumonia, abdominal infection, subcutaneous and soft tissue infection, bacteremia, and infection in various organs;
preferably, the complication includes Acute Respiratory Distress Syndrome (ARDS), sepsis, and a rise in inflammatory factors in body;
preferably, the another antibacterial agent is an agent (including chemical agents, biological agents and Chinese medicines) useful for the treatment and prevention of infection caused by Staphylococcus aureus, e.g. methicillin-resistant Staphylococcus aureus, preferably is an antibiotic, such as beta-lactam antibiotics, more preferably vancomycin, norvancomycin, teicoplanin, linezolid, daptomycin, cephapiprazole, fusidic acid, or ceftaroline.

21. A method for preventing or treating infection and complication caused by alpha-hemolysin or an alpha-hemolysin producing microorganism, including administering to a subject in need thereof the antibody or fragment thereof according to any one of claims 4 to 10, the nucleic acid molecule according to claim 11, the vector according to claim 12, the host cell according to claim 13, the conjugate or fusion protein according to claim 14 or 15, and/or the pharmaceutical composition according to claim 16, and optionally an antibacterial agent.

22. A method for diagnosing infection caused by alpha-hemolysin or an alpha-hemolysin producing microorganism, including contacting the antibody or fragment thereof according to any one of claims 4 to 10, the nucleic acid molecule according to claim 11, the vector according to claim 12, the host cell according to claim 13, the conjugate or fusion protein according to claim 14 or 15, and/or the pharmaceutical composition according to claim 16 with a sample from a subject.
